# EUROPEAN PATENT SPECIFICATION

(11) **EP 2 222 299 B1**
(45) Date of publication and mention of the grant of the patent: **23.11.2011**
(21) Application number: 08852672.8
(22) Date of filing: 27.10.2008
(51) Int. Cl.: A61K 31/445, A61K 31/451, A61P 29/00

(54) **USE OF LEVOCABASTINE FOR MODULATING GENERATION OF PRO-INFLAMMATORY CYTOKINES**
VERWENDUNG VON LEVOCABASTIN ZUR MODULIERUNG DER ERZEUGUNG VON PROINFLAMMATORISCHEN ZYTOKINEN
COMPOSITIONS ET PROCEDES DE MODULATION DE LA PRODUCTION DE CYTOKINES PRO-INFLAMMATOIRES

(30) Priority: 19.11.2007 US 988913 P
(43) Date of publication of application: 01.09.2010
(73) Proprietor: Bausch & Lomb Incorporated, Rochester, NY 14604-2701 (US)
(72) Inventor: BUCOLO, Claudio, I-95125 Catania (IT); WARD, Keith, Wayne, Ontario NY 14519 (US); ZHANG, Jinzhong, Pittsford NY 14534 (US)
(74) Representative: Glas, Holger
(86) International application number: PCT/US2008/081328
(87) International publication number: WO 2009/067317

(56) References cited:
- WO-A-97/01337
- US-A- 5 192 780
- US-A- 5 648 358
- US-A1- 2001 025 040
- US-A1- 2003 130 263
- US-B1- 6 569 443
- US-B1- 6 649 602
- MINAMI KAZUHISA ET AL: "Increasing effect by simultaneous use of levocabastine and pemirolast on experimental allergic conjunctivitis in rats" BIOLOGICAL & PHARMACEUTICAL BULLETIN, vol. 28, no. 3, March 2005 (2005-03), pages 473-476, XP002520598 ISSN: 0918-6158
- STOKES T C ET AL: "Rapid onset of action of levocabastine eye-drops in histamine-induced conjunctivitis" CLINICAL AND EXPERIMENTAL ALLERGY, vol. 23, no. 9, 1993, pages 791-794, XP009113781 ISSN: 0954-7894
- CANONICA G W ET AL: "Topical azelastine in perennial allergic conjunctivitis." CURRENT MEDICAL RESEARCH AND OPINION 2003, vol. 19, no. 4, 2003, pages 321-329, XP009114137 ISSN: 0300-7995
- PAZDRAK K ET AL: "Inhibitory effect of levocabastine on allergen-induced increase of nasal reactivity to histamine and cell influx" ALLERGY (COPENHAGEN), vol. 48, no. 8, 1993, pages 598-601, XP002520599 ISSN: 0105-4538
- BUSCAGLIA S ET AL: "Topical ocular levocabastine reduces ICAM-1 expression on epithelial cells both in vivo and in vitro" CLINICAL AND EXPERIMENTAL ALLERGY, vol. 26, no. 10, 1996, pages 1188-1196, XP002520600 ISSN: 0954-7894
- PAULY A ET AL: "Inhibition of histamine-induced human conjunctival cell release of pro-inflammatory cytokines IL-6 and IL-8" IOVS, vol. 45, no. Suppl. 2, April 2004 (2004-04), page U530, XP009113680 & ANNUAL MEETING OF THE ASSOCIATION-FOR-RESEARCH-IN-VISION-AND-OPH THALM OLOGY; FT LAUDERDALE, FL, USA; APRIL 24 -29, 2004 ISSN: 0146-0404
- YANNI J M ET AL: "A CURRENT APPRECIATION OF SITES FOR PHARMACOLOGICAL INTERVENTION IN ALLERGIC CONJUNCTIVITIS: EFFECTS OF NEW TOPICAL OCULAR DRUGS" ACTA OPHTHALMOLOGICA, XX, XX, vol. 77, no. 228, 1 January 1999 (1999-01-01), pages 33-37, XP000980225
- DECHANT K L ET AL: "LEVOCABASTINE. A REVIEW OF ITS PHARMACOLOGICAL PROPERTIES AND THERAPEUTIC POTENTIAL AS A TOPICAL ANTIHISTAMINE IN ALLERGIC RHINITIS AND CONJUNCTIVITIS" DRUGS, ADIS INTERNATIONAL LTD, vol. 41, no. 2, 1 January 1991 (1991-01-01), pages 202-224, XP000654986 ISSN: 0012-6667

## Description

### BACKGROUND

The present invention relates to compositions and methods for modulating generation of pro-inflammatory cytokines. In particular, the present invention relates to compositions that comprise levocabastine (or a salt or ester thereof) alone or in combination with other antihistamines and methods for modulating inflammation using such compositions. More particularly, the present invention relates to such compositions and methods for treating or controlling ocular diseases, disorders, or conditions that have an inflammatory component.

Ocular inflammation is characterized by redness, swelling, and/or pain association with infection, irritation, or trauma to the eye. Common triggers of ocular inflammation include allergies, meibomian gland dysfunction, ocular diseases, and ophthalmic surgical procedures.

The anterior segment of the eye (the term, as used herein, includes the anterior portion of the globe of the eye and adjacent tissues) is continuously exposed to the environment and thus presents many potential opportunities for invasion by environmental virulent pathogens. The common types of microorganisms causing ophthalmic infections are viruses, bacteria, and fungi. These microorganisms may directly invade the surface of the eye or permeate into the globe of the eye through trauma or surgery. The microorganisms may attack any part of the eye structure, including the conjunctiva, the cornea, the uvea, the vitreous body, the retina, and the optic nerve. Ophthalmic infections can cause severe pain, swollen and red tissues in or around the eye, and blurred and decreased vision.

Ophthalmic conditions may be classified as front-of-eye diseases, such as corneal edema, anterior uveitis, pterygium, corneal diseases, or opacifications with an exudative or inflammatory component, conjunctivitis, allergy- and laser-induced exudation, or back-of-eye diseases such as exudative macular degeneration, macular edema, diabetic retinopathy, age-related macular degeneration, or retinopathy of prematurity. Back-of-eye diseases comprise the largest number of causes for vision loss. There has been growing evidence that many back-of-the eye diseases have etiology in inflammation. A.M. Joussen et al., FASEB J., Vol. 18, 1450 (2004); J. Marx, Science, Vol. 311, 1704 (2006).

In addition, dry eye, also known as keratoconjunctivitis sicca ("KCS"), is a common front-of-the-eye disorder affecting millions of people each year. Dry eye conditions can be caused by a variety of factors: There has been increasing evidence that inflammation may be an important factor in the pathogenesis of KCS. For example, inflammation of the lacrimal and meibomian glands can curb tear production. In addition, elevated levels of pro-inflammatory mediators, including IL-1, IL-6, IL-8, and TNF-α, have been detected in the conjunctival tissues of patients afflicted with systemic autoimmune diseases, such as Sjögren's syndrome. These patients also suffer with severe dry eye.

Pauly et al., IOVS, vol. 45, no. Suppl. 2, 2004, investigated the effects of several H₁ receptor antagonists including, e.g, azelastine, ketotifen and levocabastine on histamine-induced release of inflammatory cytokines from human conjunctival epithelial cells. Herein, histamine increased the release of IL-6 and IL-8 in cell supernatants and all the H₁ receptor antagonists tested presented dose-dependent inhibitory effects on IL-8 production. Azelastine, ketotifen and levocabastine were found to inhibit the release of IL-6 from histamine-induced conjunctival cells in a dose-dependent manner.

Yanni et al., Acta Ophthalmologica, vol. 77, no. 228, 1999, pages 33-37 refers to studies showing that exposure of human conjunctival epithelial cells to histamine leads to the production of pro-inflammatory cytokines IL-6 and IL-8. Herein, it appears that the treatment of the epithelial cells with drugs that possess histamine H₁ antagonist properties prevents cytokine production. In this context, it is referred to the newer anti-histamines Emadine and levocabastine. It was reported that Patanol was more potent as an inhibitor of histamine-stimulated cytokine production by the epithelial cells than would be predicted from its histamine H₁ antagonist affinity.

It is informative first to discuss briefly some more important aspects of inflammation. The body's innate cascade is activated soon after invasion by a foreign pathogen begins. Leukocytes (neutrophils, eosinophils, basophils, monocytes, and macrophages) are attracted to the site of infection in an attempt to eliminate the foreign pathogen through phagocytosis. Leukocytes and some affected tissue cells are activated by the pathogens to synthesize and release pro-inflammatory cytokines such as IL-1β, IL-3, IL-5, IL-6, IL-8, IL-12, TNF-α (tumor necrosis factor-α), GM-CSF (granulocyte-macrophage colony-stimulating factor), and MCP-1 (monocyte chemotactic protein-1). These released cytokines then further attract more immune cells to the infected site, amplifying the response of the immune system to defend the host against the foreign pathogen. For example, IL-8 and MCP-1 are potent chemoattractants for, and activators of, neutrophils and monocytes, respectively, while GM-CSF prolongs the survival of these cells and increases their response to other pro-inflammatory agonists. TNF-α can activate both types of cell and can stimulate further release of IL-8 and MCP-1 from them. IL-1 and TNF-α are potent chemoattractants for T and B lymphocytes, which are activated to produce antibodies against the foreign pathogen. IL-12, which is produced by B lymphocytes, dendritic cells, monocytes, and macrophages, induces proliferation of T lymphocytes and natural killer ("NK") cells and their production of INF-γ, and enhances cytotoxicity of T lymphocytes and NK cells.

Although an inflammatory response is essential to clear pathogens from the site of infection, a prolonged or overactive inflammatory response can be damaging to the surrounding tissues. For example, inflammation causes the blood vessels at the infected site to dilate to increase blood flow to the site. As a result, these dilated vessels become leaky. After prolonged inflammation, the leaky vessels can produce serious edema in, and impair the proper functioning of, the surrounding tissues (see; e.g., V.W.M. van Hinsbergh, Arteriosclerosis, Thrombosis, and Vascular Biology, Vol. 17, 1018 (1997)). In addition, a continued dominating presence of macrophages at the injured site continues the production of toxins (such as reactive oxygen species) and matrix-degrading enzymes (such as matrix metalloproteinases) by these cells, which are injurious to both the pathogen and the host's tissues. Therefore, a prolonged or overactive inflammation should be controlled to limit the unintended damages to the body and to hasten the body's recovery process.

Glucocorticoids (also referred to herein as "corticosteroids") represent one of the most effective clinical treatment for a range of inflammatory conditions, including acute inflammation. However, steroidal drugs can have side effects that threaten the overall health of the patient.

It is known that certain glucocorticoids have a greater potential for elevating intraocular pressure ("IOP") than other compounds in this class. For example, it is known that prednisolone, which is a very potent ocular anti-inflammatory agent, has a greater tendency to elevate IOP than fluorometholone, which has moderate ocular anti-inflammatory activity. It is also known that the risk of IOP elevations associated with the topical ophthalmic use of glucocorticoids increases over time. In other words, the long-term use of these agents to treat or control persistent ocular conditions increases the risk of significant IOP elevations. In addition, use of corticosteroids is also known to increase the risk of cataract formation in a dose- and duration-dependent manner. Once cataracts develop, they may progress despite discontinuation of corticosteroid therapy.

Chronic administration of glucocorticoids also can lead to drug-induced osteoporosis by suppressing intestinal calcium absorption and inhibiting bone formation. Other adverse side effects of chronic administration of glucocorticoids include hypertension, hyperglycemia, hyperlipidemia (increased levels of triglycerides) and hypercholesterolemia (increased levels of cholesterol) because of the effects of these drugs on the body metabolic processes.

Therefore, there is a continued need to provide improved pharmaceutical compositions for modulating pro-inflammatory cytokines. It is also desirable to provide such improved compositions and methods for treating or controlling ocular diseases, conditions, or disorders having an inflammatory component.

### SUMMARY

The present invention provides a pharmaceutical composition comprising: (a) an active pharmaceutical ingredient ("API"); and (b) a pharmaceutically acceptable vehicle; wherein the API consists of: (i) levocabastine or a pharmaceutically acceptable salt or ester thereof; or (ii) levocabastine and an additional Hi-receptor antagonist, or pharmaceutically acceptable saltsor esters thereof; for use in the treatment or controlling of an inflammatory ocular disease, condition, or disorder in a patient, said disease, condition, or disorder being selected from the group consisting of dry eye, anterior uveitis, iritis, iridocyclitis, keratitis, corneal ulcer, corneal edema, sterile corneal infiltrates, anterior scleritis, episcieritis, blepharitis, post-operative (or post-surgical) ocular inflammation, posterior-segment diseases having etiology in inflammation, inflammatory sequelae of an infection, and combinations thereof.

The present invention also provides the use of an active pharmaceutical ingredient consisting of: (a) levocabastine or a pharmaceutically acceptable salt or ester thereof; or (b) levocabastine and an additional H1-receptor antagonist, or pharmaceutically acceptable salts or esters thereof; for the preparation of a medicament for the treatment or control of an inflammatory ocular disease, condition, or disorder in a patient, said disease, condition, or disorder being selected from the group consisting of dry eye, anterior uveitis, iritis, iridocyclitis, keratitis, corneal ulcer, corneal edema, sterile corneal infiltrates, anterior scleritis, episcieritis, blepharitis, postoperative (or post-surgical) ocular inflammation, posterior-segment diseases having etiology in inflammation, inflammatory sequelae of an infection, and combinations thereof.

As used herein, the term "control" also includes reduction, alleviation, amelioration, and prevention.

In general, the present invention provides pharmaceutical compositions useful for modulating generation of pro-inflammatory cytokines.

In one aspect, the present invention provides pharmaceutical compositions for use in treating or controlling ocular inflammatory diseases, conditions, or disorders in a subject in need of such treating or controlling. Such inflammatory diseases, conditions, or disorders have etiology in, or produce, inflammation.

In another aspect, a composition for use according to the present invention comprises levocabastine, a salt thereof, or an ester thereof, in an effective amount for treating or controlling a selected ocular inflammatory disease, condition, or disorder.

In still another aspect a composition for use according to the present invention comprises levocabastine, a salt thereof, or an ester thereof, in an effective amount for modulating generation ofIL-12p40, IL-8, VEGF, IL-1-ra, IL-1β, IP-10, or combinations thereof.

In still another aspect, the composition for use according to the present invention further comprises another H₁-receptor antagonist.

In yet another aspect, said another H₁-receptor antagonist is selected from the group consisting of acrivastine, cetirizine, azelastine, loratadine, desloratadine, ebastine, mizolastine, fexofenadine, olopatadine, salts thereof, esters thereof, and combinations thereof.

In another aspect, said inflammatory diseases, conditions, or disorders are of the anterior segment and include dry eye (keratoconjunctivitis sicca or KCS), anterior uveitis (including iritis and iridocyclitis), keratitis, corneal ulcer, corneal edema, sterile corneal infiltrates, anterior scleritis, episcleritis, blepharitis, and post-operative (or post-surgical) ocular inflammation resulting from procedures such as photorefractive keratectomy, cataract removal surgery, intraocular lens ("IOL") implantation, laser-assisted in situ keratomileusis ("LASIK"), conductive keratoplasty, and radial keratotomy.

In still another aspect, such inflammatory diseases, conditions, or disorders of the anterior segment result from an infection caused by bacteria, viruses, fungi, or protozoans.

In yet another aspect, said inflammatory diseases, conditions, or disorders are of the posterior segment and include diabetic retinopathy ("DR"), age-related macular degeneration ("AMD," including dry and wet AMD), diabetic macular edema ("DME"), posterior uveitis, optic neuritis, inflammatory optic neuropathy (including that caused by glaucoma), and combinations thereof.

In yet another aspect, a pharmaceutical composition for use according to the present invention comprises an ophthalmic topical formulation; injectable formulation; or implantable formulation, system, or device.

In yet another aspect, said cytokines are selected from the group consisting of IL-12p40, IL-8, VEGF, IL-1-ra, IL-1β, IP-10, and combinations thereof.

In yet another aspect, the composition for use according to the invention further comprises another H₁-receptor antagonist.

Other features and advantages of the present invention will become apparent from the following detailed description and claims and appended drawings.

### BRIEF DESCRIPTION OF THE DRAWINGS

Figure 1 shows the effect of levocabastine on interferon-inducible protein-10 (IP-10) release after 12h of TNF-α challenge. In panel A EoL-1 cells have been differentiated with PMA 24h before the experiment, whereas panel B shows the effects on undifferentiated EoL-1 cells. (** p < 0,01 vs basal; *** p < 0,001 vs basal; § p < 0,05 vs TNF- a; §§ p < 0,01 vs TNF- a.)
Figure 2 shows the effect of levocabastine on IL-1ra release after 12h of TNF-α challenge. EoL-1 cells have been differentiated with PMA 24h before the experiment. (*** p < 0.001 vs basal; §§ p < 0.01 vs TNF-α, §§§ p < 0,001 vs TNF-α.)
Figure 3 shows the effect of levocabastine on IL-1β released 12h after TNF-α challenge. EoL-1 cells have been differentiated with PMA 24h before the experiment. (* p < 0.05 vs basal; ** p < 0.001 vs basal; § p < 0.05 vs TNF-α.)
Figure 4 shows in Panel A: Levocabastine is able to reduce the release of VEGF of EoL-1 cells differentiated with PMA 24h before the experiment. Panel B: Naïve EoL-1 cells challenged with TNF-α show a reduced release of VEGF after levocabastine with cyclodextrin pre-treatment. (** p < 0.01 vs basal; § p < 0.05 vs TNF-α; §§ p < 0.01 vs TNF-α.)
Figure 5 shows in Panel A and B show the effect of levocabastine to reduce the release of IL-12p40 from, respectively, differentiated and undifferentiated EoL-1 cells 12h after TNF-α challenge. (*** p < 0.001 vs TNF-α.)
Figure 6 shows in Panel A and B show the effect of levocabastine to reduce the release of VEGF from, respectively, differentiated and undifferentiated EoL-1 cells 12h after TNF- a challenge. (* p < 0.05 vs TNF-α; ** p < 0.01 vs TNF-α.)
Figure 7 shows the effect of levocabastine on IL-12p40 release by undifferentiated EoL-1 cells. Supernatants were analysed 24h after TNF-α challenge. (*** p < 0.001 vs TNF-α.)
Figure 8 shows, in panel A, the effect of levocabastine on VEGF release by PMA differentiated EoL-1 cells, whereas panel B represents the same experiment performed on naive cells. Supernates were analysed 24h after TNF- a challenge. (* p < 0.05 vs TNF-α; ** p < 0.001 vs TNF-α.)
Figure 9 shows that levocabastine is able to reduce IL-8 release by PMA differentiated EoL-1 cells after TNF-α challenge. (*** p < 0.001 vs TNF-α.)
Figure 10 shows the effects of levocabastine on cytokine release of EoL-1 cells exposed to various ligands, after TNF-α exposure. Panel A: IL-12p40 analysis in PMA differentiated EoL-1 cells supernatants. Panel B: IL-12p40 presence in the supernatants of naïve EoL-1 cells. (* p < 0.05 vs TNF-α; ** p < 0.01 vs TNF-α; *** p < 0.001 vs TNF-α.)
Figure 11 shows, in panel A, IL-1ra analysis in PMA differentiated EoL-1 cells supernatants; Panel B, IL-1ra presence in the supernatants of naïve EoL-1 cells. (** p < 0.01 vs TNF-α; *** p < 0.001 vs TNF-α.)
Figure 12 shows that the release by non-differentiated EoL-1 cells is inhibited by levocabastine even in the presence of pro-inflammatory ligands such as VCAM-1 or fibronectin. (*** p < 0.001 vs TNF-α.)
Figure 13 shows that the IL-6 release by non-differentiated EoL-1 cells is inhibited by levocabastine even in the presence of pro-inflammatory ligands such as VCAM- or fibronectin. (*** p < 0.001 vs TNF-α.)
Figure 14 shows the level of VEGF levels in PMA differentiated and indifferentiated EoL-1 cells supernatants. Levocabastine is able to reduce VEGF release after TNF-α challenge. (*** p < 0.001 vs TNF-α.)

### DETAILED DESCRIPTION

As used herein, a soft steroid is one that has good anti-inflammatory activity and lower propensity to raise intraocular pressure. A soft drug is a biologically active drug that is metabolically unstable so that it undergoes a predictable, one-step transformation to an inactive metabolite after its pharmacologic effects have been expressed at or near the site of application. This means that these drugs are much less likely to raise intraocular pressure after administration, even in steroid responders.

In general, the present invention provides pharmaceutical compositions useful for modulating generation of pro-inflammatory cytokines.

In still another aspect a composition for use according to the present invention comprises levocabastine, a salt thereof, or an ester thereof, in an effective amount for modulating generation ofIL-12p40, IL-8, VEGF, IL-1-ra, IL-1β, IP-10, or combinations thereof.

If unregulated, these cytokines directly or indirectly can amplify the inflammatory response, resulting in excessive damage to the host tissue. For example IL-1β stimulates T cell activation by enhancing production of IL-2 and its receptor, enhances B cell proliferation and maturation, enhances NK cell cytotoxicity, induces IL-6, IL-8, TNF-α, GM-CSF, and prostaglandin E₂ production by macrophages, and is pro-inflammatory by inducing expression of chemokines, such as ICAM-1 and VCAM-1, on endothelium cells. It has been demonstrated that IL-12p40 homodimer is a potent chemoattractant for leukocyte recruitment to the airway lumen in inflammatory conditions such as asthma and respiratory viral infection. T.D. Russell et al., J. Immunol., Vol. 171, 6866 (2003). IL-8 is a chemokine that mediates chemotaxis and activation of neutrophils, induces proliferation of thymocytes, enhances mast cell growth, and induces production of leukotriene B4. See; e.g., K. Mitsuyama et al., Clin. Exp. Immunol., Vol. 96, 432 (1994); G.D. Gray et al., J Histochem. & Cytochem., Vol. 45, No. 11, 1461 (1997). Elevated expression of IL-1-ra and other cytokines (such as TNF-α, IL-1β, IL-6, IFN-γ, MCP-1, and MIP-2) were observed in the uvea and retina of uveitic rats. A.F. de Vos et al., Invest. Ophthalmol. & Vis. Sci., Vol. 35, No. 11, 3873 (1994). IP-10 (IFN-γ-inducible protein 10) is a chemoattractant for activated T cells. I. Salomon et al., J Immunol., Vol. 169,2685 (2002). VEGF is a cytokine often found at sites of inflammation and is a mediator of undesired angiogenesis in pathological conditions. In vitro, it has been found that VEGF enhanced endothelial cell expression of MCP-1 (monocyte chemoattractant protein 1) and IL-8, and in combination with IFN-γ synergistically induced endothelial cell production of the potent T cell chemoattractant IP-10. M.E.J. Reinders et al., J. Clin. Invest., Vol. 112, No. 11, 1655 (2003). Thus, an initial relative small number of cytokines can produce an amplified adverse effect in the host because of their direct interaction with each other, or indirect interaction through various cells of the immune system. Conversely, inhibition or regulation of a relatively small number of key cytokines can produce a significant positive control of the disorder or condition.

Allergic inflammation is an important pathophysiological feature of several disabilities or medical conditions including allergic asthma, atopic dematitis, allergic rhinitis and several ocular allergic diseases. Allergic reactions may generally be divided into two components; the early phase reaction, and the late phase reaction. While the contribution to the development of symptoms from each of the phases varies greatly between diseases, both are usually present and provide us a framework for understanding allergic disease.

The early phase of the allergic reaction typically occurs within minutes, or even seconds, following allergen exposure and is also commonly referred to as the immediate allergic reaction or as a Type I allergic reaction. The reaction is caused by the release of histamine and mast cell granule proteins by a process called degranulation, as well as the production of leukotrienesm protaglandins, and cytokines, by mast cells following the cross-linking of allergen specific IgE molecules bound to mast cell FcεRI receptors. These mediators affect nerve cells causing itching, smooth muscle cells causing contraction (leading to the airway narrowing seen in allergic asthma), goble cells causing mucus production, and endothelial cells causing vasodilation and edema.

The late phase reaction is also sometimes called the Type IV allergic reaction or delayed type hypersensitivity and may take as long as 6 - 12 hours to fully develop following an encounter with allergen. The products of the early phase reaction include chemokines and molecules that act on endothelial cells and cause them to express intercellular adhesion molecules ("ICAM") (such as vascular cell adhesion molecule ("VCAM") and selectins), which together result in the recruitment and activation of leukocytes from the blood into the site of the allergic reaction. Typically, the infiltrating cells observed in allergic reactions contain a high proportion of lymphocytes, and especially, of eosinophils. The recruited eosinophils will degranulate releasing a number of cytotoxic molecules (including Major Basic Protein and eosinophil peroxidase) as well as produce a number of cytokines such as IL-5. The recruited T-cells produce more cytokines, leading to further recruitment of mast cells and eosinophils, and in plasma cell isotype switching to IgE which will bind to the mast cell FcεRI receptors and prime the individual for further allergic responses. This late phase reaction constitutes the inflammatory component of allergic reactions. A composition of the present invention can control or otherwise inhibit such inflammatory component of allergic reactions by controlling or inhibiting the production or release of inflammatory cytokines and chemokines by immune cells. A composition of the present invention can provide synergistic enhanced efficacy of an anti-allergic medicament by controlling the severity of this inflammatory component through controlling or inhibitng the production of cytokines and chemokines by immune cells.

Said anti-allergic medicament comprises an anti-histamine, an anti-bradikinin, an anti-kallidin, a β₂ adrenergic receptor agonist, a leukotriene-receptor antagonist, a leukotriene-synthesis inhibitor, an anti-IgE agent, a mast cell stabilizer, an anticholinergic agent, or combinations thereof.

In still another aspect, a composition for use according to the present invention further comprises another H₁-receptor antagonist.

In yet another aspect, said another H₁-receptor antagonist is selected from the group consisting of acrivastine, cetirizine, azelastine, loratadine, desloratadine, ebastine, mizolastine, fexofenadine, olopatadine, salts thereof, esters thereof, and combinations thereof.

In one embodiment, a composition for use according to the present invention comprises: (a) levocabastine or a pharmaceutically acceptable salt or ester thereof; and (b) desloratadine or a pharmaceutically acceptable salt or ester thereof. Such a composition can modulate the generation of cytokines selected from the group consisting of IL-12p40, IL-8, VEGF, IL-1-ra, IL-1β, IP-10, IL-4, IL-6, IL-13, GM-CSF, TNF-α, RANTES, eotoxin, ICAM-1, p-selectin, and combinations thereof.

In another embodiment, a composition for use according to the present invention comprises: (a) levocabastine or a pharmaceutically acceptable salt or ester thereof; and (b) fexofenadine or a pharmaceutically acceptable salt or ester thereof. Such a composition can modulate the generation of cytokines selected from the group consisting of IL-12p40, IL-8, VEGF, IL-1-ra, IL-1β, IP-10, GM-CSF, RANTES, and combinations thereof.

In still another embodiment, a composition for use according to the present invention comprises: (a) levocabastine or a pharmaceutically acceptable salt or ester thereof; and (b) cetirizine or a pharmaceutically acceptable salt or ester thereof. Such a composition can modulate the generation of cytokines selected from the group consisting of IL-12p40, IL-8, VEGF, IL-1-ra, IL-1β, IP-10, ICAM-1, leukotriene C4, prostaglandin D2, and combinations thereof.

In still another embodiment, a composition for use according to the present invention comprises: (a) levocabastine or a pharmaceutically acceptable salt or ester thereof; and (b) olopatadine or a pharmaceutically acceptable salt or ester thereof. Such a composition can modulate the generation of cytokines selected from the group consisting of IL-12p40, IL-8, VEGF, IL-1-ra, IL-1β, IP-10, MCP-1, RANTES, and combinations thereof.

In still another embodiment, a composition for use according to the present invention comprises: (a) levocabastine or a pharmaceutically acceptable salt or ester thereof; and (b) ketotifen or a pharmaceutically acceptable salt or ester thereof. Such a composition can modulate the generation of cytokines selected from the group consisting of IL-12p40, IL-8, VEGF, IL-1-ra, IL-1β, IP-10, IL-4, TNF-α, ICAM-1, VCAM, and combinations thereof.

Ocular inflammatory pathways commence with the triggering of the arachidonic acid cascade. This cascade is triggered either by mechanical stimuli (such as the case of unavoidable surgically-inflicted trauma) or by chemical stimuli (such as foreign substances (e.g., components of disintegrated pathogenic microorganisms) or allergens). Prostaglandins are generated in most tissues by activation of the arachidonic acid pathway. Phospholipids in the damaged cell membrane are the substrate for phospholipase A to generate arachidonic acid and, in turn, the cyclooxygenase ("COX") and lipoxygenase enzymes act on arachidonic acid to produce a family of pro-inflammatory prostaglandins, thromboxanes, and leukotrienes. These pro-inflammatory compounds recruit more immune cells (such as macrophages and neutrophils) to the site of injury, which then produce a greater amount of other pro-inflammatory cytokines, including those mentioned above, and can further amplify the inflammation.

Cataract surgery with intraocular lens ("IOL") implantation and glaucoma filtering microsurgery (trabeculectomy) are among the common ophthalmic surgical operations. These procedures are usually associated with some post-operative inflammation. The use of anti-inflammatory agents post-operatively can rapidly resolve this event to relieve the patient from pain, discomfort, visual impairment, and to reduce the risk of further complications (such as the onset of cystoid macular edema).

Thus, in one aspect, the present invention provides compounds or compositions for use in treating or controlling inflammatory diseases, conditions, or disorders of the anterior segment in a subject, wherein such inflammatory diseases, conditions, or disorders result from an infection caused by bacteria, viruses, fungi, protozoans, or combinations thereof.

In another aspect, such infection comprises an ocular infection.

In another aspect, such inflammatory diseases, conditions, or disorders of the anterior segment result from the physical trauma of ocular surgery.

In still another aspect, said inflammatory diseases, conditions, or disorders of the anterior segment include dry eye, anterior uveitis (including; e.g., iritis and iridocyclitis), keratitis, corneal ulcer, corneal edema, sterile corneal infiltrates, anterior scleritis, episcleritis, blepharitis, and post-operative (or post-surgical) ocular inflammation resulting from procedures such as photorefractive keratectomy, cataract removal surgery, intraocular lens ("IOL") implantation, laser-assisted in situ keratomileusis ("LASIK"), conductive keratoplasty, and radial keratotomy.

In another aspect, said inflammatory diseases, conditions, or disorders of the posterior segment include diabetic retinopathy ("DR"), age-related macular degeneration ("AMD," including dry and wet AMD), diabetic macular edema ("DME"), posterior uveitis, optic neuritis, inflammatory optic neuropathy (including that caused by glaucoma), and combinations thereof.

In another aspect, the present invention provides an ophthalmic pharmaceutical composition for use in treating or controlling inflammatory sequelae of an infection. In one embodiment, such inflammatory sequelae comprise acute inflammation. In another embodiment, such inflammatory sequelae comprise chronic inflammation of the anterior segment of the eye. In another embodiment, such inflammatory sequelae comprise chronic inflammation of the posterior segment of the eye.

The concentration of levocabastine, another H₁-receptor antagonist, a salt thereof, or an ester thereof in a pharmaceutical composition of the present invention can be in the range from about 0.0001 to about 100 mg/ml (or, alternatively, from about 0.001 to about 50 mg/ml, or from about 0.001 to about 30 mg/ml, or from about 0.001 to about 25 mg/ml, or from about 0.001 to about 10 mg/ml, or from about 0.001 to about 5 mg/ml, or from about 0.01 to about 30 mg/ml, or from about 0.01 to about 25 mg/ml, or from about 0.01 to about 10 mg/ml, or from about 0.1 to about 10 mg/ml, or from about 0.1 to about 5 mg/ml).

In one embodiment, a composition for use according to the present invention is in a form of a suspension, dispersion, gel, or ointment. In another embodiment, the suspension or dispersion is based on an aqueous solution. For example, a composition of the present invention can comprise sterile saline solution. In still another embodiment, micrometer-or nanometer-sized particles of levocabastine, another H₁-receptor antagonist, a salt thereof, or an ester thereof can be coated with a physiologically acceptable surfactant (non-limiting examples are disclosed below), then the coated particles are dispersed in a liquid medium. The coating can keep the particles in a suspension. Such a liquid medium can be selected to produce a sustained-release suspension. For example, the liquid medium can be one that is sparingly soluble in the ocular environment into which the suspension is administered.

In another aspect, a composition for use according to the present invention can further comprise a non-ionic surfactant, such as polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, Tween® 20), poloxamers (synthetic block polymers of ethylene oxide and propylene oxide, such as those commonly known by their trade names of Pluronic®; e.g., Pluronic® F127 or Pluronic® F108)), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are delineated in Martindale, 34th ed., pp. 1411-1416 (Martindale, "The Complete Drug Reference," S. C. Sweetman (Ed.), Pharmaceutical Press, London, 2005) and in Remington, "The Science and Practice of Pharmacy," 21st Ed., p. 291 and the contents of chapter 22, Lippincott Williams & Wilkins, New York, 2006. The concentration of a non-ionic surfactant, when present, in a composition of the present invention can be in the range from about 0.001 to about 5 weight percent (or alternatively, from about 0.01 to about 4, or from about 0.01 to about 2, or from about 0.01 to about 1, or from about 0.01 to about 0.5 weight percent).

In addition, a composition for use according to the present invention can include additives such as buffers, diluents, carriers, adjuvants, or other excipients. Any pharmacologically acceptable buffer suitable for application to the eye may be used. Other agents may be employed in the composition for a variety of purposes. For example, buffering agents, preservatives, co-solvents, oils, humectants, emollients, stabilizers, or antioxidants may be employed. Water-soluble preservatives which may be employed include sodium bisulfite, sodium bisulfate, sodium thiosulfate, benzalkonium chloride, chlorobutanol, thimerosal, ethyl alcohol, methylparaben, polyvinyl alcohol, benzyl alcohol, and phenylethyl alcohol. These agents may be present in individual amounts of from about 0.001 to about 5% by weight (preferably, about 0.01% to about 2% by weight). Suitable water-soluble buffering agents that may be employed are sodium carbonate, sodium borate, sodium phosphate, sodium acetate, sodium bicarbonate, etc., as approved by the United States Food and Drug Administration ("US FDA") for the desired route of administration. These agents may be present in amounts sufficient to maintain a pH of the system of between about 2 and about 11. As such, the buffering agent may be as much as about 5% on a weight to weight basis of the total composition. Electrolytes such as, but not limited to, sodium chloride and potassium chloride may also be included in the formulation.

In one aspect, the pH of the composition is in the range from about 4 to about 11. Alternatively, the pH of the composition is in the range from about 5 to about 9, from about 6 to about 9, or from about 6.5 to about 8, or from about 5 to about 6.5. In another aspect, the composition comprises a buffer having a pH in one of said pH ranges.

In another aspect, the composition has a pH of about 7. Alternatively, the composition has a pH in a range from about 7 to about 7.5.

In still another aspect, the composition has a pH of about 7.4.

In yet another aspect, a composition also can comprise a viscosity-modifying compound designed to facilitate the administration of the composition into the subject or to promote the bioavailability in the subject. In still another aspect, the viscosity-modifying compound may be chosen so that the composition is not readily dispersed after being administered into the vitreous. Such compounds may enhance the viscosity of the composition, and include, but are not limited to: monomeric polyols (such as glycerol, propylene glycol, or ethylene glycol); polymeric polyols (such as polyethylene glycol): various polymers of the cellulose family (such as hydroxypropylmethyl cellulose ("HPMC"). carboxymethyl cellulose ("CMC") sodium, hydroxypropyl cellulose ("HPC")); polysaccharides, such as hyaluronic acid and its salts, chondroitin sulfate and its salts, dextrans (such as dextran 70), galactomannans (such as guar or hydroxypropyl guar); water soluble proteins, such as gelatin; vinyl polymers, such as, polyvinyl alcohol, polyvinylpyrrolidone, povidone; carbomers, such as carbomer 934P, carbomer 941, carbomer 940, or carbomer 974P; and acrylic acid polymers. In general, a desired viscosity can be in the range from about 1 to about 1,000 centipoises ("cps") or mPa.s.

In one aspect, a composition for use according to the invention further includes a pharmaceutically acceptable carrier. In some embodiments, the carrier comprises a physiologically acceptable buffer. In some other embodiments, the carrier can comprise a saline solution. In still other embodiments, the carrier can comprise a hydrophobic medium, such as a pharmaceutically acceptable oil for imparting a slow release of the active ingredient in a hydrophilic environment.

Non-limiting examples of physiological buffers include, but are not limited to, a phosphate buffer or a Tris-HCl buffer (comprising tris(hydroxymethyl)aminomethane and HCl). For example, a Tris-HCl buffer having pH of 7.4 comprises 3 g/l of tris(hydroxymethyl)aminomethane and 0.76 g/l of HCl. In yet another aspect, the buffer is 10X phosphate buffer saline ("PBS") or 5X PBS solution.

Other buffers also may be found suitable or desirable in some circumstances, such as buffers based on HEPES (N-{2-hydroxyethyl}peperazine-N'-{2-ethanesulfonic acid}) having pKₐ of 7.5 at 25 °C and pH in the range of about 6.8-8.2; BES (N,N-bis{2-hydroxyethyl}2-aminoethanesulfonic acid) having pKₐ of 7.1 at 25°C and pH in the range of about 6.4-7.8; MOPS (3-{N-morpholino}propanesulfonic acid) having pKₐ of 7.2 at 25°C and pH in the range of about 6.5-7.9; TES (N-tris{hydroxymethyl}-methyl-2-aminoethanesulfonic acid) having pKₐ of 7.4 at 25°C and pH in the range of about 6.8-8.2: MOBS (4-{N-morpholino}butanesulfonic acid) having pKₐ of 7.6 at 25°C and pH in the range of about 6.9-8.3; DIPSO (3-(N,N-bis{2-hydroxyethyl}amino)-2-hydroxypropane)) having pKₐ of 7.52 at 25°C and pH in the range of about 7-8.2; TAPSO (2-hydroxy-3{tris(hydroxymethyl)methylamino}-1-propanesulfonic acid)) having pKₐ of 7.61 at 25°C and pH in the range of about 7-8.2; TAPS ({(2-hydroxy-1,1-bis(hydroxymethyl)ethyl)amino}-1-propanesulfonic acid)) having pKₐ of 8.4 at 25°C and pH in the range of about 7.7-9.1; TABS (N-tris(hydroxymethyl)methyl-4-aminobutanesulfonic acid) having pKₐ of 8.9 at 25°C and pH in the range of about 8.2-9.6; AMPSO (N-(1,1-dimethyl-2-hydroxyethyl)-3-amino-2-hydroxypropanesulfonic acid)) having pKₐ of 9.0 at 25°C and pH in the range of about 8.3-9.7; CHES (2-cyclohexylamino)ethanesulfonic acid) having pKₐ of 9.5 at 25°C and pH in the range of about 8.6-10.0; CAPSO (3-(cyclohexylamino)-2-hydroxy-1-propanesulfonic acid) having pKₐ of 9.6 at 25°C and pH in the range of about 8.9-10.3; or CAPS (3-(cyclohexylamino)-1-propane sulfonic acid) having pKₐ of 10.4 at 25°C and pH in the range of about 9.7-11.1.

In certain embodiments, a composition of the present invention is formulated in a buffer having an acidic pH, such as from about 4 to about 6.8, or alternatively, from about 5 to about 6.8. In such embodiments, the buffer capacity of the composition desirably allows the composition to come rapidly to a physiological pH after being administered into the patient.

It should be understood that the proportions of the various components or mixtures in the following examples may be adjusted for the appropriate circumstances.

### EXAMPLE I

The amounts shown in Table 1 were mixed thoroughly for at least 15 minutes in a sterilized vessel. The mixture was then packaged into vials for use to treat ocular inflammation.

**Table 1**

| Ingredient | Amount |
|---|---|
| Levocabastine hydrochloride | 0.0543 g |
| Hydroxypropyl-β-cyclodextrin | 7. 5 g |
| Sodium dihydrogen phosphate di-hydrate | 0.153 g |
| Di-sodium phosphate dodecahydrate | 0.64 g |
| Sodium chloride | 0.453 g |
| Purified water | q.s. to 100 g |

### EXAMPLE 2

Two mixtures I and II are made separately by mixing the ingredients listed in Table 2. Five parts (by weight) of mixture I are mixed with twenty parts (by weight) of mixture II for 15 minutes or more. The pH of the combined mixture is adjusted to 6.2-6.4 using 1 N NaOH or 1 N HCl solution to yield a composition of the present invention.

**Table 2**

| Ingredient | | Amount |
|---|---|---|
| Mixture I | | |
| | Levocabastine HCl | 0.06 g |
| | Carbopol 934P NF | 0.25 g |
| | Purified water | 99.55 g |

| Mixture II | | |
|---|---|---|
| | Propylene glycol | 5 g |
| | EDTA | 0.1 mg |
| | Desloratadine | 0.06 g |

### EXAMPLE 3: (Reference example)

Two mixtures I and II are made separately by mixing the ingredients listed in Table 3. Five parts (by weight) of mixture I are mixed with twenty parts (by weight) of mixture II for 15 minutes or more. The pH of the combined mixture is adjusted to 6.2-6.4 using 1 N NaOH or 1 N HCl solution to yield a composition of the present invention.

**Table 3**

| Ingredient | | Amount |
|---|---|---|
| Mixture I | | |
| | Levocabastine HCl | 0.05 g |
| | Diclofenac | 0.2 g |
| | Carbopol 934P NF | 0.25 g |
| | Purified water | 99.25 g |

| Mixture II | | |
|---|---|---|
| | Propylene glycol | 5 g |
| | EDTA | 0.1 mg |
| | Fexofenadine | 0.05 g |

### EXAMPLE 4:

Two mixtures I and II are made separately by mixing the ingredients listed in Table 4. Five parts (by weight) of mixture I are mixed with twenty parts (by weight) of mixture II for 15 minutes or more. The pH of the combined mixture is adjusted to 6.2-6.4 using 1 N NaOH or 1 N HCl solution to yield a composition of the present invention.

**Table 4**

| Ingredient | | Amount |
|---|---|---|
| Mixture I | | |
| | Levocabastine HCl | 0.1 g |
| | Cetirizine | 0.1 g |
| | Carbopol 934P NF | 0.25 g |
| | Purified water | 99.35 g |

| Mixture II | | |
|---|---|---|
| | Propylene glycol | 3 g |
| | Triacetin | 7g |
| | Loteprednol etabonate | 0.1 g |
| | EDTA | 0.1 mg |

### EXAMPLE 5: (Reference example)

Two mixtures I and II are made separately by mixing the ingredients listed in Table 5. Five parts (by weight) of mixture I are mixed with twenty parts (by weight) of mixture II for 15 minutes or more. The pH of the combined mixture is adjusted to 6.2-7.5 using 1 N NaOH or 1 N HCl solution to yield a composition of the present invention.

**Table 5**

| Ingredient | | Amount |
|---|---|---|
| Mixture I | | |
| | Tobramycin sulfate | 0.3 g |
| | Levocabastine | 0.1 g |
| | Carbopol 934P NF | 0.25 g |
| | Olive oil | 99.15 g |

| Mixture II | | |
|---|---|---|
| | Propylene glycol | 7 g |
| | Glycerin | 3 g |
| | Deslotaradine | 0.1 g |
| | Cyclosporine A | 0.5 g |
| | HAP (30%) | 0.5 mg |
| | Polyhexamethylene biguanide ("PHMB") | 1-2 ppm |

| | | |
|---|---|---|
| Note: "HAP" denotes hydroxyalkyl phosphonates, such as those known under the trade name Dequest®. | | |

### EXAMPLE 6: (Reference example)

The ingredients listed in Table 6 are mixed together for at least 15 minutes. The pH of the mixture is adjusted to 6.2-7.5 using 1 N NaOH or 1 N HCl solution to yield a composition of the present invention.

**Table 6**

| Ingredient | Amount (% by weight) |
|---|---|
| Povidone | 1 |
| HAP (30%) | 0.05 |
| Glycerin | 3 |
| Propylene glycol | 3 |
| Levocabastine HCl | 0.1 |
| Trifluridine | 0.1 |
| Tyloxapol | 0.25 |
| BAK | 10-100 ppm |
| Purified water | q.s. to 100 |

| | |
|---|---|
| Note: "BAK" denotes benzalkonium chloride. | |

### EXAMPLE 7: (Reference example)

The ingredients listed in Table 7 are mixed together for at least 15 minutes. The pH of the mixture is adjusted to 7-7.5 using 1 N NaOH or 1 N HCl solution to yield a composition of the present invention.

**Table 7**

| Ingredient | Amount (% by weight) |
|---|---|
| Povidone | 1.5 |
| HAP (30%) | 0.05 |
| Glycerin | 3 |
| Propylene glycol | 3 |
| Levocabastine HCl | 0.15 |
| Foscavir | 0.1 |
| Tyloxapol | 0.25 |
| PHMB | 1-2 ppm |
| Purified water | q.s. to 100 |

### EXAMPLE 8: (Reference example)

The ingredients listed in Table 8 are mixed together for at least 15 minutes. The pH of the mixture is adjusted to 6.5-7.8 using 1 N NaOH or 1 N HCl solution to yield a composition of the present invention.

**Table 8**

| Ingredient | Amount (% by weight) |
|---|---|
| CMC (MV) | 0.5 |
| HAP (30%) | 0.05 |
| Glycerin | 3 |
| Propylene glycol | 3 |
| Levocabastine HCl | 0.08 |
| Amphotericin B | 0.05 |
| Ketorolac | 0.1 |
| Tyloxapol (a surfactant) | 0.25 |
| PHMB | 1-2 ppm |
| Purified water | q.s. to 100 |

### EXAMPLE 9: (Reference example)

The ingredients listed in Table 9 are mixed together for at least 15 minutes. The pH of the mixture is adjusted to 6.2-7.4 using 1 N NaOH or 1 N HCl solution to yield a composition of the present invention.

**Table 9**

| Ingredient | Amount (% by weight) |
|---|---|
| CMC (MV) | 0.5 |
| HAP (30%) | 0.05 |
| Glycerin | 3 |
| Propylene glycol | 3 |
| Levocabastine HCl | 0.15 |
| Miconazole | 0.1 |
| 15-deoxy-Δ-12,14-prostaglandin J2 | 0.2 |
| Tyloxapol (a surfactant) | 0.25 |
| PHMB | 1-2 ppm |
| Purified water | q.s. to 100 |

### EXAMPLE 10: (Reference example)

The ingredients listed in Table 10 are mixed together for at least 15 minutes. The pH of the mixture is adjusted to 6.2-6.8 using 1 N NaOH or 1 N HCl solution to yield a composition of the present invention.

**Table 10**

| Ingredient | Amount (% by weight) |
|---|---|
| CMC (MV) | 0.5 |
| HAP (30%) | 0.05 |
| Glycerin | 3 |
| Propylene glycol | 3 |
| Levocabastine HCl | 0.1 |
| Bacitracin zinc | 0.1 |
| Flurbiprofen | 0.1 |
| Levofloxacin | 0.1 |
| Tyloxapol (a surfactant) | 0.25 |
| PHMB | 1-2 ppm |
| Purified water | q.s. to 100 |

### EXAMPLE 11: (Reference example)

The ingredients listed in Table 11 are mixed together for at least 15 minutes. The pH of the mixture is adjusted to 6.2-6.8 using 1 N NaOH or 1 N HCl solution to yield a composition of the present invention.

**Table 11**

| Ingredient | Amount (% by weight) |
|---|---|
| CMC (MV) | 0.5 |
| HAP (30%) | 0.05 |
| Glycerin | 3 |
| Propylene glycol | 3 |
| Levocabastine HCl | 0.1 |
| Ebastine | 0.1 |
| 15-deoxy-Δ-12,14-prostaglandin J2 | 0.2 |
| Clotrimazole | 0.2 |
| Tyloxapol (a surfactant) | 0.25 |
| PHMB | 1-2 ppm |
| Purified water | q.s. to 100 |

### EXAMPLE 12: (Reference example)

The ingredients listed in Table 12 are mixed together for at least 15 minutes. The pH of the mixture is adjusted to 6.2-7 using 1 N NaOH or 1 N HCl solution to yield a composition of the present invention.

**Table 12**

| Ingredient | Amount |
|---|---|
| Ketorolac | 0.2 g |
| Levocabastine HCl | 0.2 g |
| Carbopol 934P NF | 0.25 g |
| Propylene glycol | 5 g |
| EDTA | 0.5 mg |
| Purified water | 98.65 g |

In another aspect, a composition for use according to the invention comprises levocabastine or a pharmaceutically acceptable salt or ester thereof, and a H₁-receptor antagonists other than levocabastine or its pharmaceutically acceptable salts or esters incorporated into a formulation for topical administration or periocular injection to a portion of the anterior segment. An injectable formulation can desirably comprise a carrier that provides a sustained-release of the active ingredients, such as for a period longer than about 1 week (or longer than about 1, 2, 3, 4, 5, or 6 months). In certain embodiments, the sustained-release formulation desirably comprises a carrier that is insoluble or only sparingly soluble in the anterior- or posterior-segment environment. Such a carrier can be an oil-based liquid, emulsion, gel, or semisolid. Non-limiting examples of oil-based liquids include castor oil, peanut oil, olive oil, coconut oil, sesame oil, cottonseed oil, corn oil, sunflower oil, fish-liver oil, arachis oil, and liquid paraffin.

In one embodiment, a composition of the present invention designed for topical administration, such as an eye drop, may be administered, for example, in one drop daily or multiple times daily, or two or more drops once daily or multiple times daily, or as necessary for treating or controlling the particular condition, as directed by a skilled physician.

In another embodiment, such inflammation is a long-term inflammation. In still another embodiment, such inflammation requires at least two weeks for resolution, if untreated.

In another aspect, a composition for use according to the invention the present invention is administered periocularly or in the anterior chamber. In still another aspect, a composition of the present invention is incorporated into an ophthalmic implant system or device, and the implant system or device is surgically implanted periocularly or in a tissue adjacent to the anterior portion of the eye of the patient for the sustained release of the active ingredient or ingredients. A typical implant system or device suitable for use in a method of the present invention comprises a biodegradable matrix with the active ingredient or ingredients impregnated or dispersed therein. Non-limiting examples of ophthalmic implant systems or devices for the sustained-release of an active ingredient are disclosed in U.S. Patents 5,378,475; 5,773,019; 5,902,598; 6,001,386; 6,051,576; and 6,726,918; which are incorporated herein by reference.

In yet another aspect, a composition for use according to the invention the present invention is administered once a week, once a month, once a year, twice a year, four times a year, or at a suitable frequency that is determined to be appropriate for treating or controlling an anterior-segment inflammatory disease, condition, or disorder.

### TESTING: Demonstration of Modulation of Generation of Certain Cytokines by a Present Formulation

### Cell Culture

EoL-1 (human eosinophilic leukaemia cell line) cells (Saito et al 1985; Mayumi, 1992) were maintained in RPMI-1640 medium with L-glutamine supplemented with 10% (v/v) FBS at 37 °C in a humidified atmosphere with 5% CO₂. Where indicated, 24 h before the experiment 25 ng/mL PMA ((phorbol 12-myristate 13-acetate, purchased from Sigma Aldrich) was added to the medium to induce eosinophil granulation and differentiation (Ohtsu et al., 1993; Zimmermann et al., 2000).

### Cytokine Assays

Half-million cells were aliquoted per point in a 24 well plate; each experiment was performed in triplicate and carried out in parallel with eosinophils differentiated and non differentiated with PMA (25 ng/mL, 24 h). Cells were suspended in low serum medium (RPMI-1640, 0,1% (v/v) FBS). TNF-α was used to induce cytokine secretion as previously described by Steube et al. (2000), and the net release was obtained by comparison to the basal (non- TNF-α treated). In the first experiment we evaluated the concentration and time relation between TNF-α stimulation and cytokine release. Therefore, 5, 10, and 25 ng/mL of TNF-α was administered to the cells at 0, ½, 1, 2, 3, 6, 12 and 24 h (data not shown). An aliquot of 150 µL of supernate was collected for cytokines' analysis. Then, we considered whether levocabastine, an anti-allergic drug that demonstrated to be active not only as an H₁ receptor antagonist, could affect the release of these cell mediators. Differentiated and non-differentiated EoL-1 cells were exposed from 0.1 to 2.3 mM levocabastine (Levocabastine 0.05% solution eye drops containing cyclodextrins, without benzalkonium chloride), and aliquots of the supernates were collected after 12 and 24 h. The content of cytokines at time zero was estimated by treating the cells with 400 nM calcimycin (A-23187), which is a cytolytic agent that frees all the mediators from the cytoplasmic compartment. In addition, for each experiment we tested the effect of the vehicle, which was added to the wells in a concentration equal to the maximum amount used for drug dilution.

Samples (supemates 12 and 24 h) in triplicate were analyzed using Luminex 200™ (Luminex, Austin, TX) and Beadview software v1.0 (Upstate Cell Signaling Solutions, Temecula, CA).

### Data analysis

All data are presented as mean ± SEM for the indicated number of experiments. Statistical significance was determined by Newman-Keuls test after ANOVA using GraphPad Prism (version 3.0; GraphPad Software Inc., San Diego, CA, USA). P-values < 0.05 were considered to be significant.

### Effect of levocabastine on cytokine release

Cytokines are autocrine and paracrine mediators that support inflammation acting on the vascular epithelium and modulating the activity of resident and circulating white blood cells. We evaluated the ability of levocabastine to reduce cytokine release from differentiated and non-differentiated (immature phenotype) EoL-1 cells using TNF-a as a proinflammatory stimulus. Table T-1 and Table T-2 summarize the effects of levocabastine on 13 different cytokines at 12 and 24 h after TNF-α challenge; cells were or were not exposed to PMA, as indicated.

**Table T-1**

| Detection of Cytokines After 12 hours | | | | | | |
|---|---|---|---|---|---|---|
| Treatment Cytokine | TNF-α (positive Ctrl) vs Basal | Vehicle vs Basal | TNF-α vs Lev 0.1 mM | TNF-α vs Lev 0.5 mM | TNF-α vs Lev 1.0 mM | TNF-α vs Lev 2.3 mM |
| Fractalkine (PMA) | ns | p<0.05 | ns | ns | ns | ns |
| Fractalkine (NO PMA) | ns | p<0.01 | ns | ns | ns | ns |
| IL-1α (PMA) | p<0.05 | p<0.01 | ns | ns | ns | ns |
| IL-1α (NO PMA) | p<0.05 | P<0.001 | ns | ns | ns | ns |
| IL-1β (PMA) | ns | p<0.001 | ns | ns | ns | ns |
| IL-1β (NO PMA) | ns | ns | ns | ns | ns | ns |
| IL-1ra (PMA) | p<0.001 | p<0.001 | p<0.01 | p<0.001 | p<0.001 | ns |
| IL-1ra (NO PMA) | p<0.001 | p<0.001 | ns | ns | ns | ns |
| IL-5 (PMA) | ns | p<0,05 | ns | ns | ns | ns |
| IL-5 (NO PMA) | ns | ns | ns | ns | ns | ns |
| IL-7 (PMA) | p<0.05 | p<0.01 | ns | ns | ns | ns |
| IL-7 (NO PMA) | p<0.001 | p<0.001 | ns | ns | ns | ns |
| IL-8 (PMA) | p<0.01 | p<0.01 | ns | ns | ns | ns |
| IL-8 (NO PMA) | ns | p<0.001 | ns | ns | ns | ns |
| IP-10 (PMA) | p<0.01 | p<0.001 | ns | p<0.01 | p<0.05 | ns |
| IP-10 (NO PMA) | p<0.01 | p<0.001 | ns | ns | ns | ns |
| MCP-1 (PMA) | p<0.01 | p<0.01 | ns | ns | ns | ns |
| MCP-1 (NO PMA) | p<0.001 | p<0.001 | ns | ns | ns | ns |
| MIP-1α (PMA) | ns | p<0.01 | concentrahon dependent increase of cytokine secretion p<0.001 | | | |
| MIP-1α (NO PMA) | p<0.05 | p<0.001 | concentration dependent increase of cytokine secretion p<0.01 | | | |
| MIP-1β (PMA) | p<0.001 | p<0.001 | ns | ns | ns | ns |
| MIP-1β (NO PMA) | p<0.001 | p<0.001 | ns | ns | ns | ns |
| RANTES (PMA) | p<0.001 | p<0.001 | ns | ns | ns | ns |
| RANTES (NO PMA) | p<0.05 | p<0.001 | increase of cytokine secretion p<0.001 | | | |
| VEGF (PMA) | ns | ns | ns | ns | p<0.05 | p<0.01 |
| VEGF (NO PMA) | ns | ns | ns | ns | ns | ns |

| | | | | | | |
|---|---|---|---|---|---|---|
| One-way analysis of variance (ANOVA) with Newman-Keuls post hoc test was used to compare all the pairs of treatments. ns = non significant. All the values shown are intended as decrements, except where otherwise stated. | | | | | | |

**Table T-2**

| Detection of Cytokines After 24 hours | | | | | | |
|---|---|---|---|---|---|---|
| Treatment Cytokine | TNF-α (positive ctri) vs Basal | Vehicle vs Basal | TNF-α vs Lev 0.1 mM | TNF-α vs Lev 0.5 mM | TNF-α vs Lev 1.0 mM | TNF-α vs Lev 2.3 mM |
| Fractalkine (PMA) | ns | ns | ns | ns | ns | p<0.01 |
| Fractalkine (NO PMA) | ns | p<0.001 | ns | ns | ns | ns |
| IL-1α (PMA) | ns | p<0.01 | ns | ns | ns | ns |
| IL-1α (NO PMA) | p<0.01 | p<0.001 | ns | ns | ns | ns |
| IL-1β (PMA) | p<0.01 | p<0.05 | p<0.05 | ns | p<0.05 | ns |
| IL-1β (NO PMA) | ns | ns | ns | ns | ns | ns |
| IL-1ra (PMA) | p<0.01 | p<0.05 | ns | ns | ns | ns |
| IL-1ra (NO PMA) | p<0.001 | p<0.01 | ns | ns | ns | ns |
| IL-5 (PMA) | ns | ns | ns | ns | ns | ns |
| IL-5 (NO PMA) | ns | ns | ns | ns | ns | ns |
| IL-7 (PMA) | ns | ns | ns | ns | ns | ns |
| IL-7 (NO PMA) | p<0.05 | p<0.01 | ns | ns | ns | ns |
| IL-8 (PMA) | ns | ns | ns | ns | ns | ns |
| IL-8 (NO PMA) | p<0.01 | p<0.01 | ns | ns | ns | ns |
| IP-10 (PMA) | p<0.01 | p<0.001 | ns | ns | ns | ns |
| IP-10 (NO PMA) | p<0.001 | p<0.001 | p<0.05 | p<0.05 | ns | ns |
| MCP-1 (PMA) | p<0.01 | p<0.001 | ns | ns | ns | ns |
| MCP-1 (NO PMA) | p<0.001 | p<0.001 | ns | ns | ns | ns |
| MIP-1α (PMA) | p<0.01 | p<0.01 | ns | ns | ns | ns |
| MIP-1α (NO PMA) | p<0.01 | p<0.001 | Increase in cytokine release in a concentration dependent way p<0.001 | | | |
| MIP-1β (PMA) | p<0.001 | ns | ns | ns | ns | ns |
| MIP-1β (NO PMA) | p<0.001 | p<0.001 | ns | ns | ns | ns |
| RANTES (PMA) | ns | ns | ns | ns | ns | ns |
| RANTES (NO PMA) | ns | p<0.05 | Increase in cytokine release in a concentration dependent way p<0.001 | | | |
| VEGF(PMA) | ns | ns | ns | ns | p<0.05 | p<0.01 |
| VEGF (NO PMA) | p<0.01 | ns | ns | ns | ns | p<0.01 |

| | | | | | | |
|---|---|---|---|---|---|---|
| One-way analysis of variance (ANOVA) with Newman-Keuls post hoc test was used to compare all the pairs of treatments. ns = non significant. All the values shown are intended as decrements, except where otherwise stated. | | | | | | |

EoL-1 cells were exposed to the vehicle in the same amount used to obtain the highest concentration of the drug. Interestingly, levocabastine was able to significantly reduce the release of the proinflammatory cytokine IL-1β and of IP-10, that is know to promote rapid transendothelial migration of effector cells of the immune system (Manes et al., 2006).

Furthermore, we report a very important perturbation from the vehicle, which seems to stimulate cytokine release by itself. The placebo group, in fact, produced levels of cytokines similar to the one measured using calcimycin, which induces the release of all the vescicular content of the cells by lysis. Since vehicle did not show toxicity, we speculate that this could be due to its content of cyclodextrins which interfere with the plasma membrane and, specifically, with integrin functionality (Green, 1999; Pande, 2000; Berg, 2007).

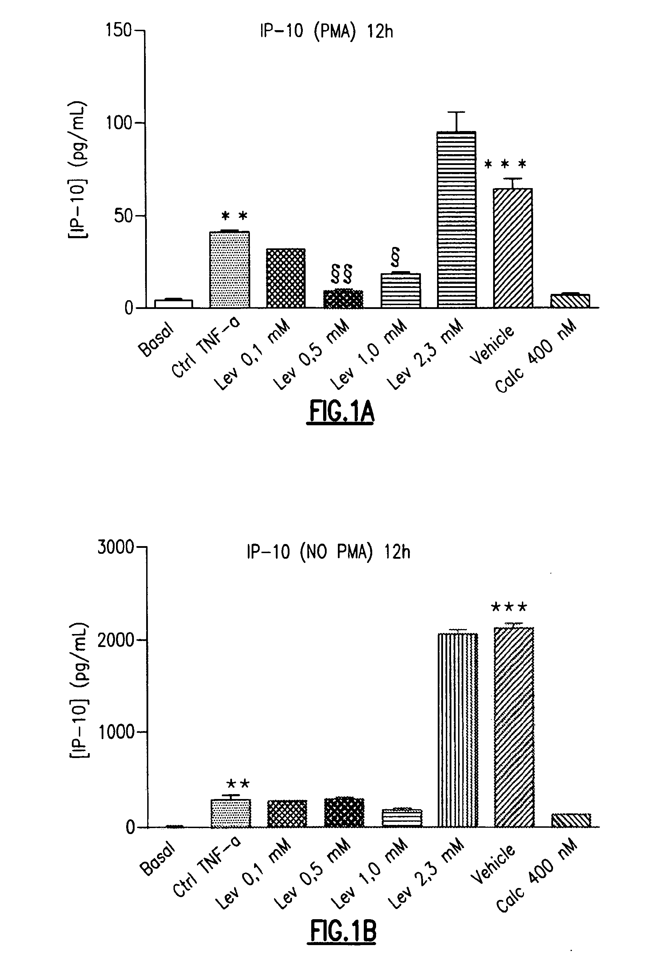

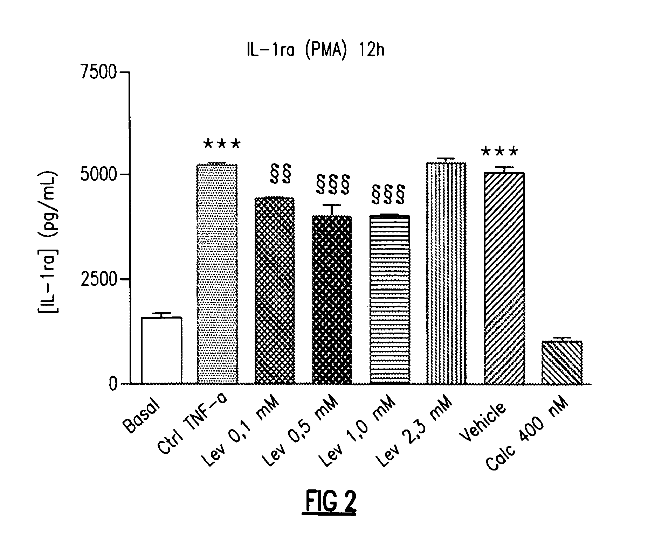

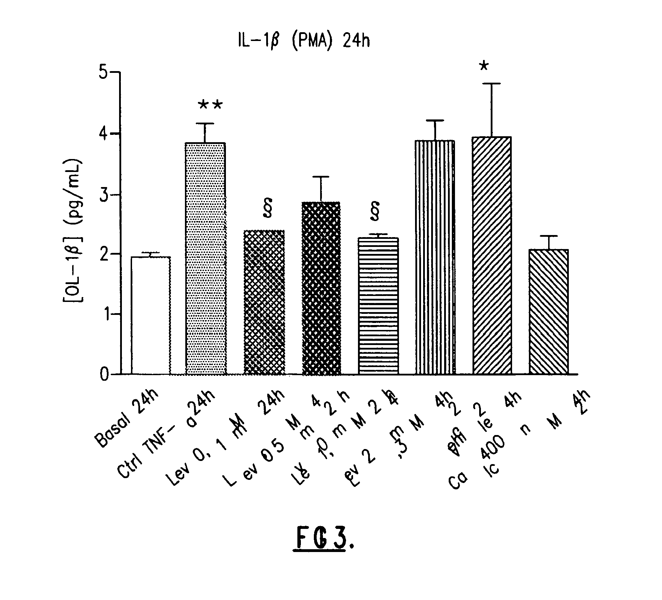

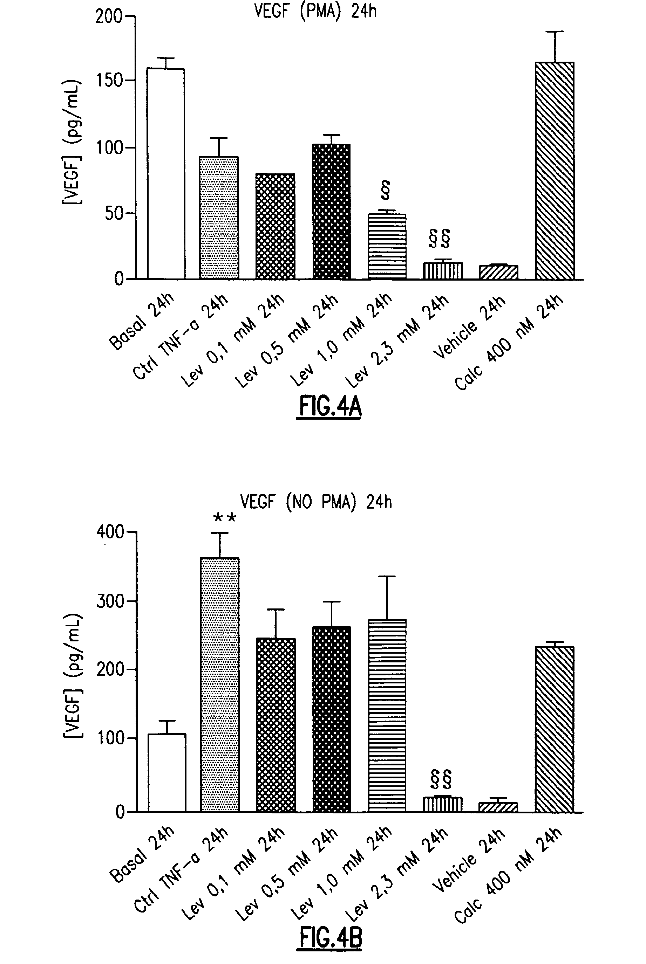

An analysis of the data summarized in Table T-1 and Table T-2 indicates that levocabastine - at three different concentrations: 0.1, 0.5 and 1.0 mM - is capable to prevent the release of the following cytokines induced by TNF-α:
IP-10 in cells exposed for 12 h without PMA or differentiated with it (see Fig. 1). This cytokine is involved in inflammatory processes (Inukal Y et al, 2007).
IL-1-ra in cells exposed for 12 and differentiated with PMA (see Fig. 2). This cytokine seems to act as an antagonist of the inflammatory cytokine IL-1 and this may be a controversial result. Although, it should be pointed out that the effect of levocabastine was observed only after 12 h of exposure in cells treated with PMA.
IL-1β in cells exposed for 12 h in cells differentiated with PMA (see Fig. 3). This cytokine is involved in the inflammatory response (Hallsworth et al 1998; Wong et al 2007). Levocabastine was effective in the PMA-treated group of cells exposed for 12 h.
VEGF in cells exposed for 24 h in cells without PMA or differentiated with it (see Fig. 4). This cytokine is relevant for the inflammatory response in eosinophils (Solomon et al. 2003; Puxeddu et al., 2005). Surprisingly, the release of this cytokine induced by TNF-α was blocked by levocabastine; however, the vehicle alone (contrary to what observed for the other cytokines) did not influence the release of VEGF (see Fig. 4).

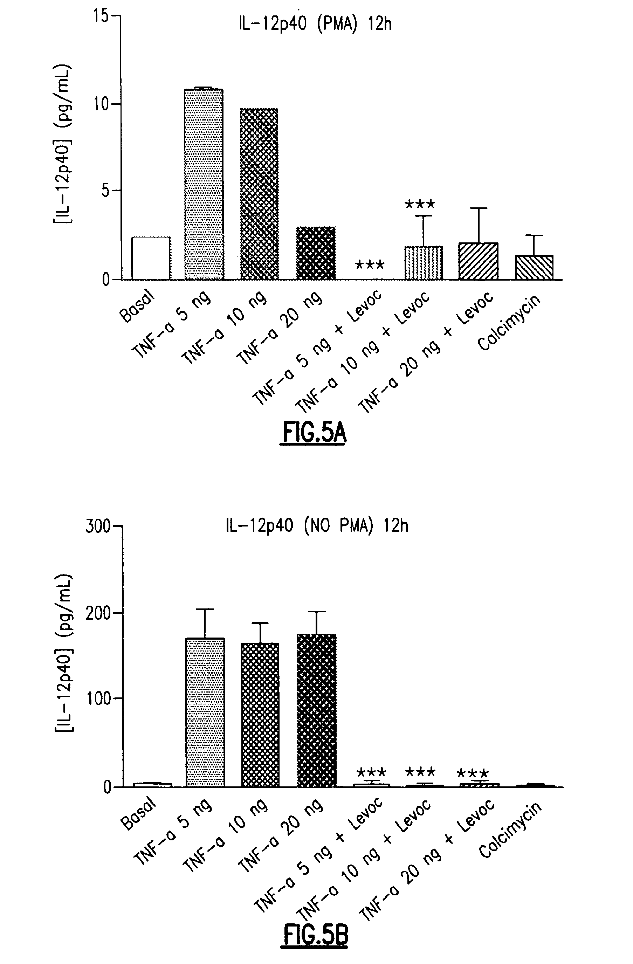

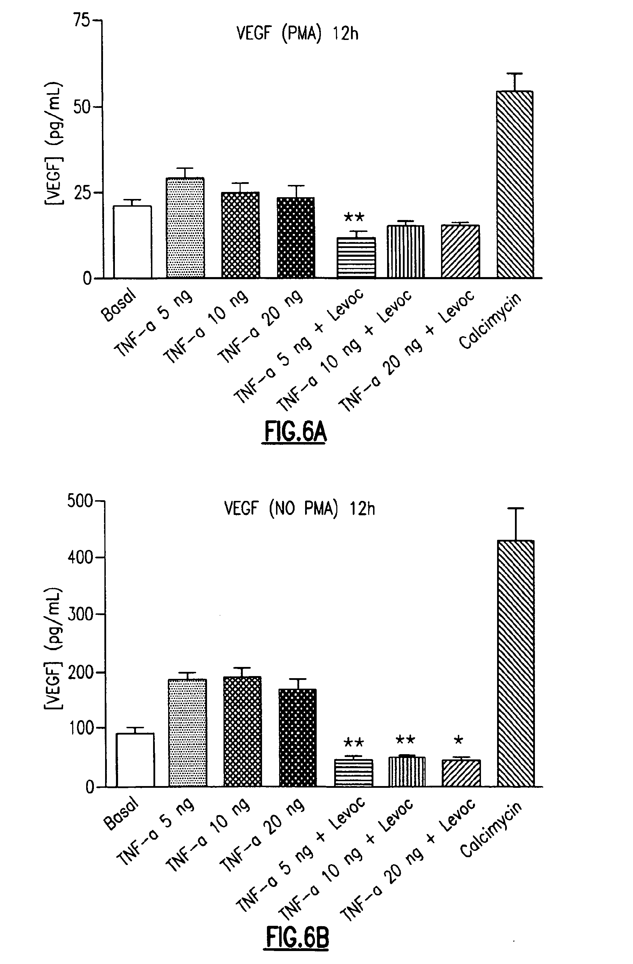

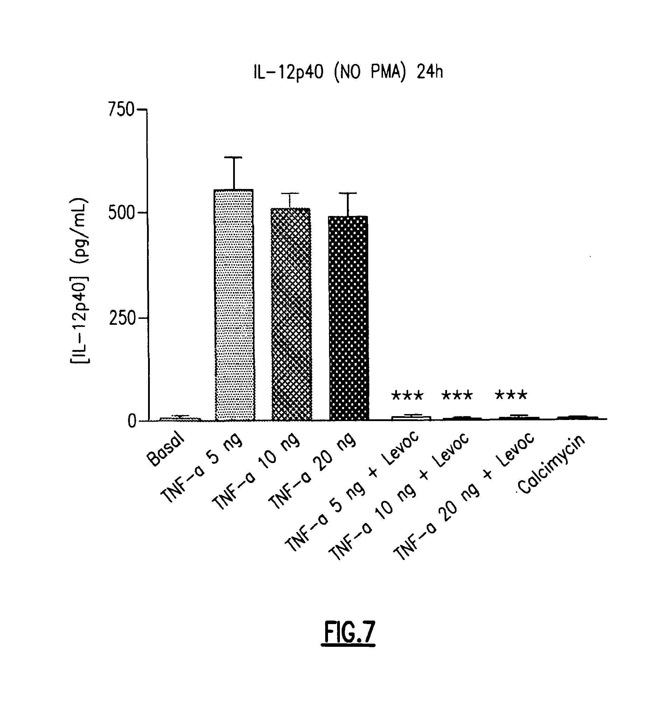

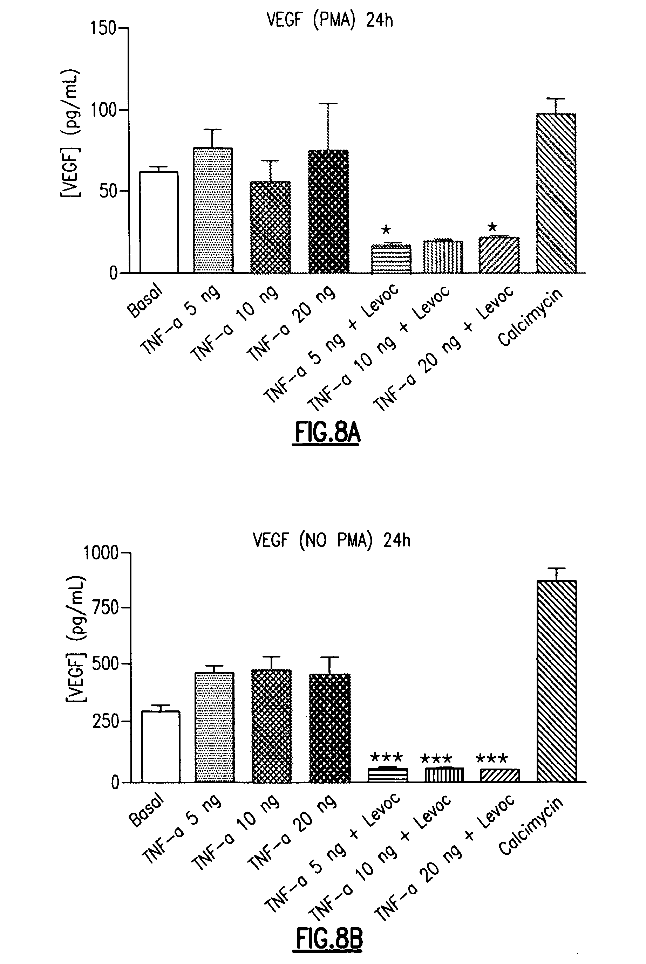

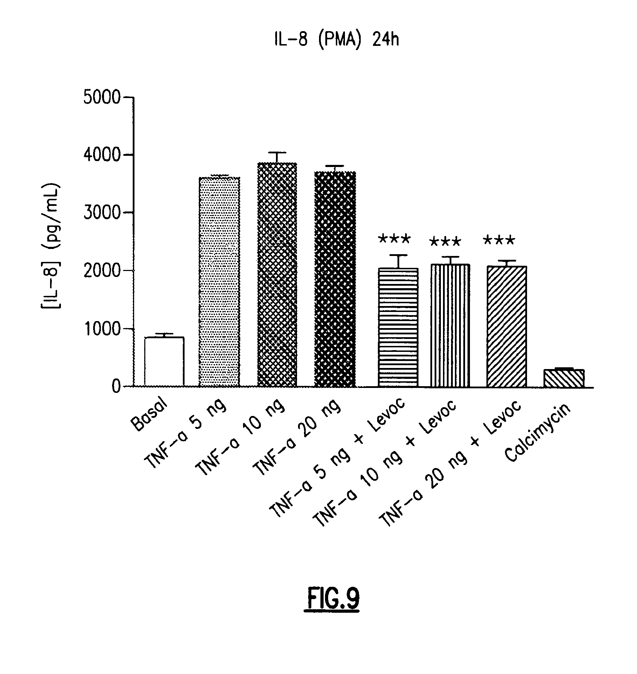

An analysis of the data summarized in Table T-3 and Table T-4 indicates that levocabastine at the fixed concentration of 2 mM is capable of reducing the release of the following cytokines induced by three different concentrations of TNF-α (5, 10 and 20 ng):
IL-12 P40 in cells exposed for 12 h without PMA or differentiated with it (see Fig. 5). This cytokine is involved in the inflammatory response in eosinophils (Wen et 1. 2006).
VEGF in cells exposed for 24 h without PMA or differentiated with it (see Fig. 6).
IL-12-P40 in cells exposed for 24 h without PMA (see Fig. 7).
VEGF in cells exposed for 24 h without PMA or differentiated with it (see Fig. 8).
IL-8 in cells exposed for 24 h in cells differentiated with PMA (see Fig. 9). This cytokine is crucial for the inflammatory response in allergic diseases (Silvestri et al. 2006).

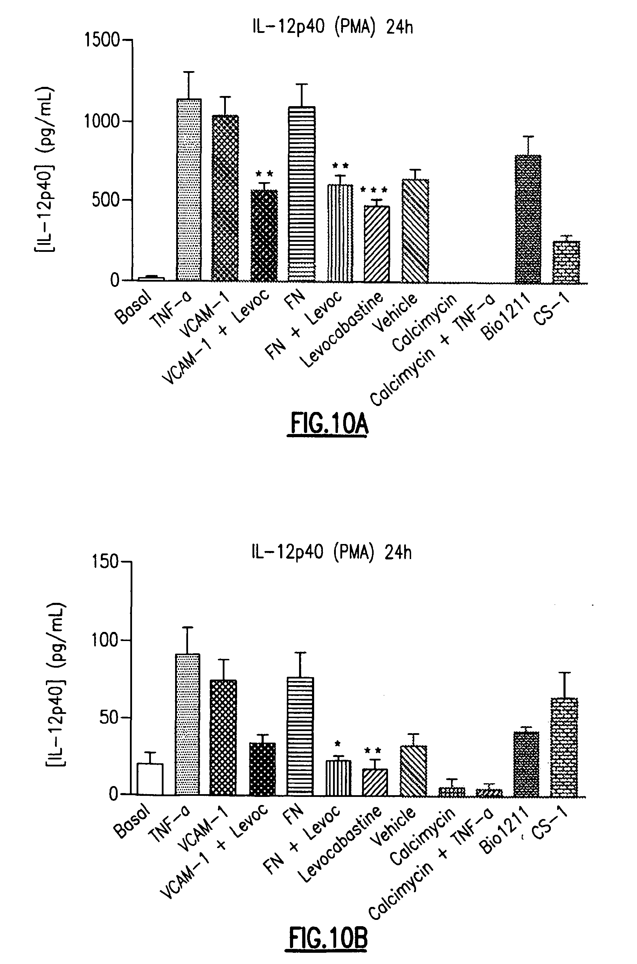

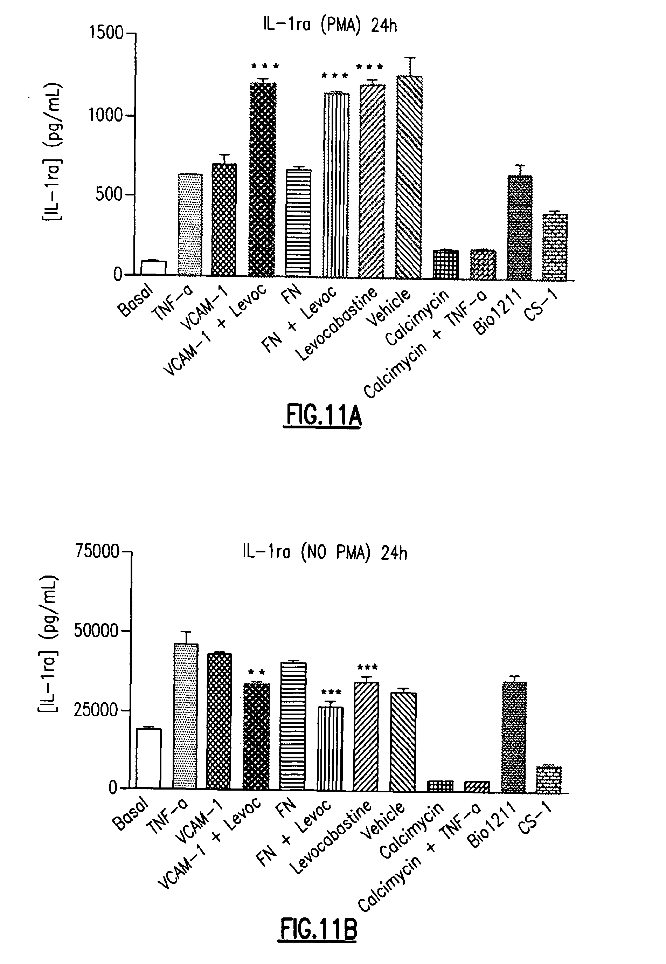

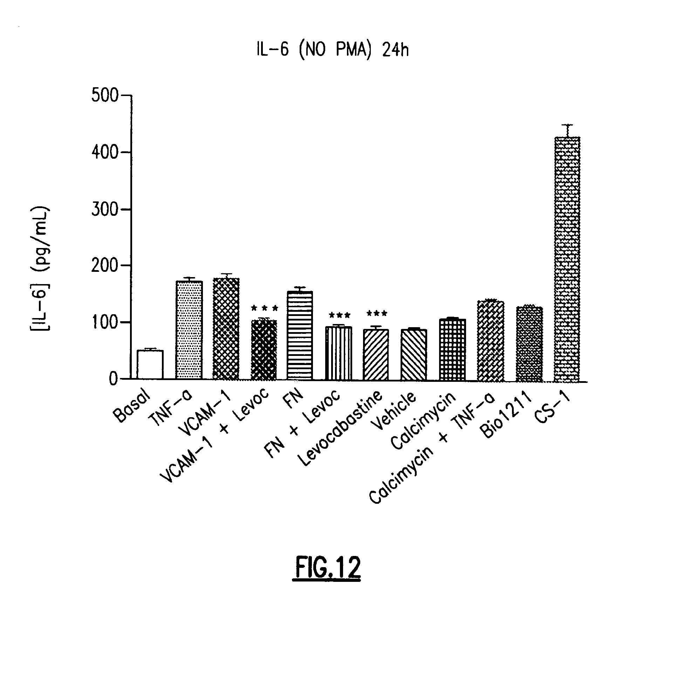

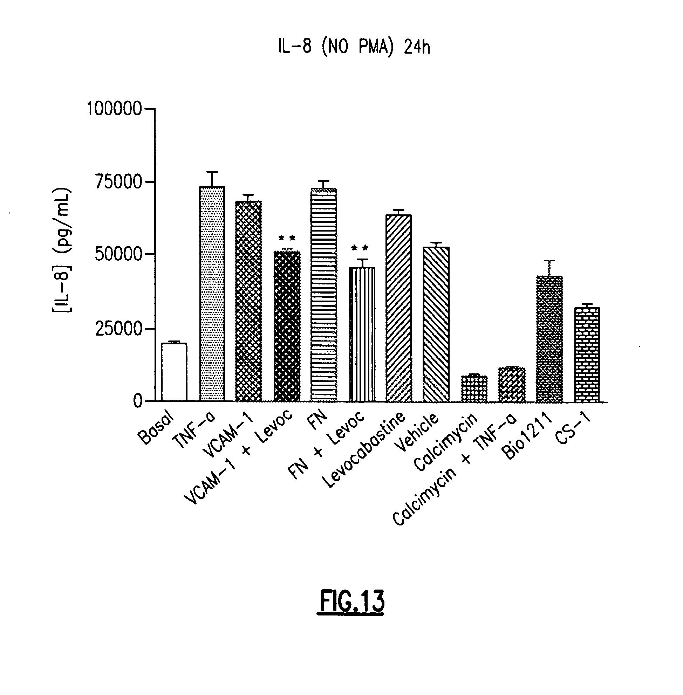

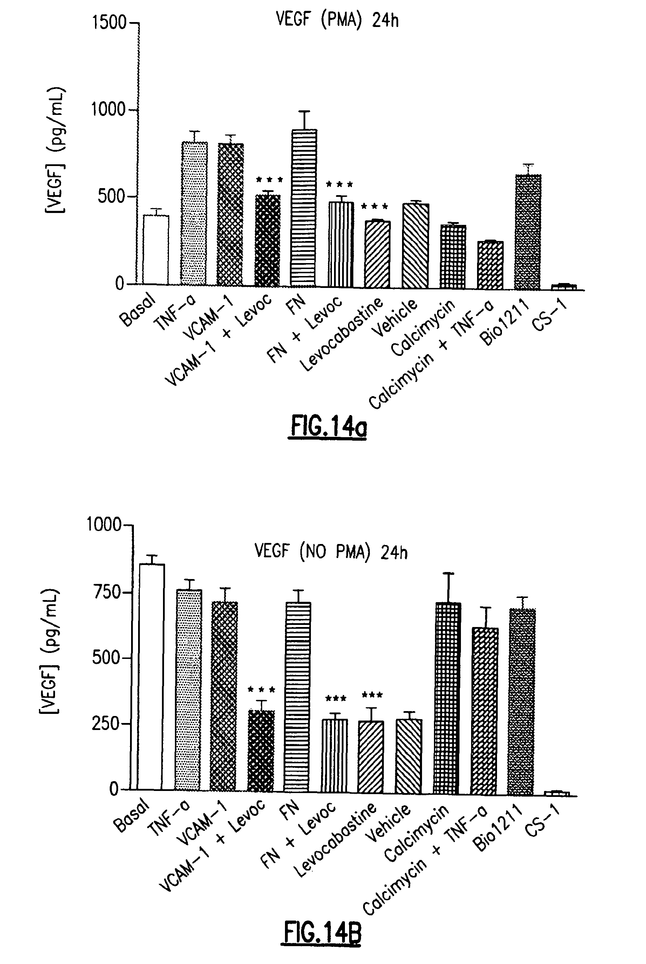

An analysis of the data summarized in Table T-5 indicates that levocabastine (2 mM) is effective in reducing the release of the following cytokines induced by TNF-α (10 ng) for 24 h. This effect is not influenced by VCAM-1 or fibronectin:
IL-12p40 in cells without PMA or differentiated with it (see Fig. 10).
IL-1-ra in cells did not exposed to PMA (see Fig. 11).
IL-6 in cells were not exposed to PMA (see Fig. 12). Another cytokine relevant for the allergic response (Gazizadeh, 2007; Fritz et al. 2006).
IL-8 in cells not exposed to PMA (see Fig. 13).
VEGF in cells cultured without PMA or differentiated with it (see Fig. 14).

The cytokines produced have an important role in stimulating the subsequent immune response and shaping its development. Suppression of their release and adhesion molecule expression in the conjunctiva, can inhibit activation and local infiltration of immune cells, and, thus limit the severity of inflammation. Therefore, we tested the ability of levocabastine to reduce the release of different cytokines, through the analysis of PMA differentiated and undifferentiated EoL-1 cell supernatants, following TNF-α stimulation. We verified a general effect of concentration-related reduction of cytokine release caused by levocabastine in this cell line. The analysis of the data related to the cytokine release has shown clearly that levocabastine is capable to cause, in Eol-1 cells, a statistical significant reduction of TNF-α-induced release of the following cytokines: IL-12p40, IL-8, VEGF. Furthermore, levocabastine significantly reduced the release of IL1-ra, IL-1β, IP-10 in a concentration-dependent manner, though increasing the secretion of MIP-1α and RANTES in a concentration-dependent way.

**Table T-3**

| Effect of Levocabastine at 2 mM on Cytokine Production After 12 hours | | | | | | |
|---|---|---|---|---|---|---|
| Treatment Cytokine | TNF-α 5ng vs Basal | TNF-α 10ng vs Basal | TNF-α 20ng vs Basal | TNF-α 5ng + Levocabastine 2mM vs TNF-α | TNF-α 10ng + Levocabastine 2mM vs TNF-α | TNF-α 20ng + Levocabastine 2mM vs TNF-α |
| Fractalkine (PMA) | ns | ns | ns | ns | ns | ns |
| Fractalkine (NO PMA) | ns | ns | ns | increase of cytokine secretion | | |
| G-CSF (PMA) | ns | ns | ns | ns | ns | ns |
| G-CSF (NO PMA) | ns | ns | ns | increase of cytokine secretion | | |
| GM-CSF (PMA) | P < 0.01 | P < 0.01 | P < 0.01 | ns | ns | ns |
| GM-CSF (NO PMA) | ns | ns | ns | ns | ns | ns |
| IL-10 (PMA) | ns | ns | ns | ns | ns | ns |
| IL-10 (NO PMA) | ns | ns | ns | ns | ns | ns |
| IL-12p40 (PMA) | P < 0.001 | P < 0.001 | ns | P < 0.001 | P < 0.001 | P < 0.001 |
| IL-12p40 (NO PMA) | P < 0.001 | P < 0.001 | P < 0.001 | P < 0.001 | P < 0.001 | P < 0.001 |
| IL-1α (PMA) | P < 0.001 | P < 0.001 | P < 0.001 | increase of cytokine secretion | | |
| IL-1α (NO PMA) | P < 0.001 | P < 0.001 | P < 0.001 | increase of cytokine secretion | | |
| IL-1β (PMA) | ns | ns | ns | ns | ns | ns |
| IL-1β (NO PMA) | ns | ns | ns | ns | ns | ns |
| IL-1ra (PMA) | P < 0.001 | P < 0.001 | P < 0.001 | increase of cytokine secretion | | |
| IL-1ra (NO PMA) | P < 0.001 | P < 0.001 | P < 0.001 | increase of cytokine secretion | | |
| IL-5 (PMA) | ns | ns | ns | ns | ns | ns |
| IL-5 (NO PMA) | ns | ns | ns | ns | ns | ns |
| IL-6 (PMA) | ns | ns | ns | ns | ns | ns |
| IL-6 (NO PMA) | ns | ns | ns | ns | ns | ns |
| IL-7 (PMA) | P < 0.001 | P < 0.001 | P < 0.001 | ns | ns | ns |
| IL-7 (NO PMA) | P < 0.001 | P < 0.001 | P < 0.001 | ns | ns | ns |
| IL-8 (PMA) | P < 0.001 | P < 0.001 | P < 0.001 | ns | ns | ns |
| IL-8 (NO PMA) | P < 0.001 | P < 0.001 | P < 0.001 | ns | ns | ns |
| IP-10 (PMA) | P < 0.001 | P < 0.001 | P < 0.001 | ns | ns | ns |
| IP-10 (NO PMA) | P < 0.001 | P < 0.001 | P < 0.001 | increase of cytokine secretion | | |
| MCP-1 (PMA) | ns | ns | ns | ns | ns | ns |
| MCP-1 (NO PMA) | P < 0.01 | P < 0.001 | P < 0.001 | ns | ns | ns |
| MIP-1α (PMA) | ns | ns | ns | ns | ns | ns |
| MIP-1α (NO PMA) | P < 0.001 | P < 0.001 | P < 0.001 | increase of cytokine secretion | | |
| MIP-1β (PMA) | P < 0.001 | P < 0.001 | P < 0.001 | increase of cytokine secretion | | |
| MIP-1β (NO PMA) | P < 0.001 | P < 0.001 | P < 0.001 | increase of cytokine secretion | | |
| RANTES (PMA) | P < 0.01 | P < 0.01 | P < 0.01 | increase of cytokine secretion | | |
| RANTES (NO PMA) | P < 0.001 | P < 0.001 | P < 0.001 | increase of cytokine secretion | | |
| TGF-α (PMA) | ns | ns | ns | ns | ns | ns |
| TGF-α (NO PMA) | ns | ns | ns | ns | ns | ns |
| VEGF (PMA) | ns | ns | ns | P < 0.01 | ns | ns |
| VEGF (NO PMA) | P < 0.05 | P < 0.05 | P < 0.05 | P < 0.01 | P < 0.01 | P < 0.05 |

**Table T-4**

| Effect of Levocabastine at 2 mM on Cytokine Production at 24 hours | | | | | | |
|---|---|---|---|---|---|---|
| Treatment Cytokine | TNF-α 5ng vs Basal | TNF-α 10ng vs Basal | TNF-α 20ng vs Basal | TNF-α 5ng + Levocabastine 2mM vs TNF-α | TNF-α 10ng + Levocabastine 2mM vs TNF-α | TNF-α 20ng + Levocabastine 2mM vs TNF-α |
| Fractalkine (PMA) | ns | ns | ns | ns | ns | ns |
| Fractalkine (NO PMA) | ns | ns | ns | increase of cytokine secretion | | |
| G-CSF (PMA) | ns | ns | ns | ns | ns | ns |
| G-CSF (NO PMA) | ns | ns | ns | increase of cytokine secretion | | |
| GM-CSF (PMA) | ns | ns | ns | ns | ns | ns |
| GM-CSF (NO PMA) | ns | ns | ns | ns | ns | ns |
| IL-10 (PMA) | ns | ns | ns | ns | ns | ns |
| IL-10 (NO PMA) | ns | ns | ns | ns | ns | ns |
| IL-12p40 (PMA) | ns | ns | ns | ns | ns | ns |
| IL-12p40 (NO PMA) | P < 0.001 | P < 0.001 | P < 0.001 | P < 0.001 | P < 0.001 | P < 0.001 |
| IL-1α (PMA) | ns | ns | P < 0.01 | increase of cytokine secretion | | |
| IL-1α (NO PMA) | P < 0.05 | P < 0.05 | P < 0.05 | increase of cytokine secretion | | |
| IL-1β (PMA) | ns | ns | ns | ns | ns | ns |
| IL-1β (NO PMA) | ns | ns | ns | ns | ns | ns |
| IL-1ra (PMA) | P < 0.001 | P < 0.001 | P < 0.001 | increase of cytokine secretion | | |
| IL-1ra (NO PMA) | P < 0.001 | P < 0.001 | P < 0.001 | P < 0.01 | P < 0.05 | P < 0.01 |
| IL-5 (PMA) | ns | ns | ns | ns | ns | ns |
| IL-5 (NO PMA) | ns | ns | ns | ns | ns | ns |
| IL-6 (PMA) | ns | ns | ns | ns | ns | ns |
| IL-6 (NO PMA) | ns | ns | ns | ns | ns | ns |
| IL-7 (PMA) | ns | ns | P < 0.05 | ns | ns | ns |
| IL-7 (NO PMA) | P < 0.01 | P < 0.01 | P < 0.01 | ns | ns | ns |
| IL-8 (PMA) | P < 0.001 | P < 0.001 | P < 0.001 | P < 0.001 | P < 0.001 | P < 0.001 |
| IL-8 (NO PMA) | P < 0.001 | P < 0.001 | P < 0.001 | ns | ns | ns |
| IP-10 (PMA) | P < 0.001 | P < 0.001 | P < 0.001 | ns | ns | ns |
| IP-10 (NO PMA) | P < 0.001 | P < 0.01 | P < 0.001 | increase of cytokine secretion | | |
| MCP-1 (PMA) | ns | ns | ns | ns | ns | ns |
| MCP-1 (NO PMA) | P < 0.01 | P < 0.01 | P < 0.001 | ns | ns | ns |
| MIP-1α (PMA) | ns | P < 0.05 | P < 0.01 | ns | ns | ns |
| MIP-1α (NO PMA) | ns | ns | ns | increase of cytokine secretion | | |
| MIP-1β (PMA) | P < 0.001 | P < 0.001 | P < 0.001 | ns | ns | ns |
| MIP-1β (NO PMA) | P < 0.001 | P < 0.001 | P < 0.001 | increase of cytokine secretion | | |
| RANTES (PMA) | P < 0.001 | P < 0.001 | P < 0.001 | increase of cytokine secretion | | |
| RANTES (NO PMA) | P < 0.01 | P < 0.01 | P < 0.01 | increase of cytokine secretion | | |
| TGF-α (PMA) | ns | ns | ns | ns | ns | ns |
| TGF-α (NO PMA) | P < 0.05 | ns | P < 0.05 | P < 0.05 | ns | P < 0.05 |
| VEGF (PMA) | ns | ns | ns | P<0.05 | ns | P < 0.05 |
| VEGF (NO PMA) | P < 0.05 | P < 0.05 | P < 0.05 | P < 0.001 | P < 0.001 | P < 0.001 |

**Table T-5**

| Effect of Levocabastine 2 mM on Cytokine Production in the Presence or Absence of VCAM-1 or Fibronectin After 24 hours | | | | | | | | | |
|---|---|---|---|---|---|---|---|---|---|
| Treatment Cytokine | TNF-α 10 ng vs Basal | VCAM-1 vs Basal | VCAM-1 + Levocabastine 2mM vs VCAM-1 | FN vs Basal | FN + Levocabastme 2mM vs FN | Levocabastin 2mM vs TNF-α | Vehicle vs Basal | Bio1211 vs TNF-α | CS-1 vs TNF-α |
| Fractalkine (PMA) | ns | ns | ns | ns | Increase | ns | Increase | ns | ns |
| Fractalkine (NO PMA) | ns | ns | ns | ns | ns | ns | ns | p < 0.05 | P < 0.001 |
| G-CSF (PMA) | ns | ns | Increase | ns | Increase | ns | Increase | ns | ns |
| G-CSF (NO PMA) | ns | ns | ns | ns | ns | ns | ns | ns | ns |
| GM-CSF (PMA) | ns | ns | ns | ns | ns | ns | ns | ns | ns |
| GM-CSF (NO PMA) | P < 0.001 | P < 0 001 | ns | P < 0 001 | ns | ns | Increase | P < 0.05 | P < 0.001 |
| IL-10 (PMA) | ns | ns | ns | ns | ns | ns | ns | ns | ns |
| IL-10 (NO PMA) | ns | ns | ns | ns | ns | ns | ns | ns | P < 0.001 |
| IL-12p40 (PMA) | P < 0.001 | P < 0.001 | P<001 | p < 0.001 | P<0.01 | P < 0 001 | Increase | P < 0.05 | P < 0.001 |
| IL-12p40 (NO PMA) | P<0.01 | P < 0.05 | ns | P < 0.05 | P < 0.05 | P < 0.01 | ns | P < 0.05 | ns |
| IL-1α (PMA) | P < 0.001 | P < 0.001 | Increase | P < 0.001 | Increase | Increase | Increase | P < 0.05 | ns |
| IL-1α (NO PMA) | ns | ns | ns | ns | ns | ns | ns | ns | ns |
| IL-1β (PMA) | ns | ns | ns | ns | ns | ns | ns | ns | ns |
| IL-1β (NO PMA) | P < 0001 | P < 005 | ns | P < 0.001 | ns | ns | Increase | ns | ns |
| IL-1 ra (PMA) | P < 0 001 | P < 0.001 | Increase | P < 0.001 | Increase | Increase | Increase | ns | P < 0.01 |
| IL-1ra (NO PMA) | P < 0.001 | P < 0 001 | P < 001 | p < 0.001 | P < 0.001 | P < 0.001 | Increase | P < 0.001 | P < 0.001 |
| IL-5 (PMA) | ns | ns | ns | ns | ns | ns | ns | ns | ns |
| IL-5 (NO PMA) | ns | ns | ns | ns | ns | ns | ns | ns | ns |
| IL-6 (PMA) | ns | ns | ns | ns | ns | ns | ns | ns | Increase |
| IL-6 (NO PMA) | P < 0.001 | P < 0.001 | P < 0.001 | P < 0.001 | P < 0.001 | P < 0.001 | Increase | P < 0.05 | P < 0.001 |
| IL-7 (PMA) | P < 0.001 | P < 0.001 | Increase | P < 0.001 | Increase | ns | Increase | ns | ns |
| IL-7 (NO PMA) | P < 0.001 | P < 0.001 | ns | P < 0 001 | ns | ns | Increase | ns | P < 0.01 |
| IL-8 (PMA) | ns | ns | Increase | P < 0.05 | Increase | Increase | Increase | ns | Increase |
| IL-8 (NO PMA) | P < 0.001 | P < 0.001 | P < 0.001 | P < 0.001 | P < 0.001 | ns | Increase | P < 0.001 | P < 0.001 |
| IP-10 (PMA) | P < 0.001 | P < 0.001 | ns | P < 0 001 | ns | ns | Increase | P < 0.001 | ns |
| IP-10 (NO PMA) | P < 0.001 | P < 0 001 | ns | P < 0.001 | ns | ns | Increase | P < 0.001 | ns |
| MCP-1 (PMA) | P < 0.001 | P < 0.001 | Increase | P < 0.001 | Increase | ns | Increase | P < 0.001 | P < 0 001 |
| MCP-1 (NO PMA) | P < 0.001 | P < 0.001 | ns | P < 0.001 | Increase | Increase | Increase | ns | P < 0.001 |
| MIP-1α (PMA) | ns | ns | Increase | ns | Increase | Increase | ns | ns | ns |
| MIP-1α (NO PMA) | P < 0.001 | P < 0.001 | Increase | P < 0.001 | Increase | Increase | Increase | ns | Increase |
| MIP-1β (PMA) | P < 0.001 | P < 0.001 | Increase | P < 0.001 | Increase | Increase | Increase | ns | P < 0.001 |
| MIP-1β (NO PMA) | P < 0.001 | P < 0 001 | ns | P < 0.001 | ns | ns | Increase | ns | Increase |
| RANTES (PMA) | ns | ns | Increase | ns | Increase | Increase | ns | ns | ns |
| RANTES (NO PMA) | ns | ns | Increase | ns | Increase | Increase | Increase | ns | ns |
| TGF-α (PMA) | P < 0.05 | P < 0.05 | ns | P < 0.05 | ns | ns | ns | ns | P < 0.05 |
| TGF-α (NO PMA) | P < 0.01 | P < 0.05 | ns | P < 0.05 | ns | ns | P < 0.001 | ns | P < 0.001 |
| VEGF (PMA) | P < 0.001 | P < 0.001 | P<0001 | P < 0.001 | P<0001 | P < 0.001 | ns | P < 0.05 | P < 0.001 |
| VEGF (NO PMA) | ns | ns | P < 0.001 | ns | P < 0.001 | P < 0.001 P | P < 0.001 | ns P | P < 0.001 |

| | | | | | | | | | |
|---|---|---|---|---|---|---|---|---|---|
| Note: FN = fibronectin; Bio1211 (4-((2-methylphenyl)aminocarbonyl)-aminophenyl)acetyl-Leu-Asp-Val-Pro-OH) is peptidic ligand for α₄β₁ (also known as VLA-4) integrin, available from Biogen. Inc., Cambridge. Massachusetts (see; e.g., J. Chiba et al.. Bioorg. Med. Chem. Lett., Vol. 15, 41 (2005)): CS-1 is a peptide representing the major cell adhesion domain in the type II connecting segment of fibronectin (see; e.g., A.C.H.M. van Dinther-Janssen et al., Ann. Rheumatic Diseases, Vol. 52, 672 (1993)). Biol21 and CS-1 were purchased from Weil am Rhein, Germany, and used as positive controls. | | | | | | | | | |

Thus, the present work shows that levocabastine, an H₁-receptor antagonist, can reduce the production of several important pro-inflammatory cytokines, and thus, can find utility in the treatment of inflammatory diseases. It should be understood that the utility and optimal concentration and/or dose of levocabastine may be determined for specific disorder in question based on this work.

### REFERENCES

Berg KA, Zardeneta G, Hargreaves KM, Clarke WP, Milam SB. Integrins regulate opioid receptor signaling in trigeminal ganglion neurons. Neuroscience, 2007 Feb 9; 144(3):889-97. Epub 2006 Dec 8.
Buscaglia S, Paolieri F, Catrullo A, Fiorino N, Riccio AM, Pesce G, Montagna P, Bagnasco M, Ciprandi G, Canonica GW. Topical ocular levocabastine reduces ICAM-1 expression on epithelial cells both in vivo and in vitro. Clin Exp Allergy, 1996 Oct; 26(10):1188-96.
Fritz DK, Kerr C, Tong L, Smyth D, Richards CD. Oncostatin-M up-regulates VCAM-1 and synergizes with IL-4 in eotaxin expression: involvement of STAT6. The Journal of Immunology, 2006; 176: 4352-4360.
Ghazizadeh M. Essential role of IL-6 signaling pathway in keloid pathogenesis. J Nippon Med Sch. 2007; 74: 11-22.
Green JM, Zhelesnyak A, Chung J, Lindberg FP, Sarfati M, Frazier WA, Brown EJ. Role of cholesterol in formation and function of a signaling complex involving alphavbeta3, integrin-associated protein (CD47), and heterotrimeric G proteins. J Cell Biol. 1999 Aug. 9; 146(3):673-82.
Hallsworth MP, Soh CPC, Twort CHC, Lee TH, Hirst SJ. Cultured human airway smooth muscle cells stimulated by interleukin-1β enhance eosinophil survival. Am. J. Respir. Cell Mol. Biol. 1998; 19:910-919.
Inukai Y, Momobayashi A, Sugawara N, Aso Y. Changes in expression of T-helper (Th)1- and Th2-associated chemokine receptors on peripheral lymphocytes and plasma concentrations of their ligands, interferon-inducible protein-10 and thymus and activation-regulated chemokine, after antithyroid drug administration in hyperthyroid patients with Graves' disease. Eur. J. Endocrinol. 2007; 156: 623-630.
Izushi K., Nakahara H., Tai N., Nio M., Watanabe T., Kamei C. The role of histamine H1 receptors in late-phase reaction of allergic conjunctivitis. Eur. J. Pharmacol. 2002; 440:79-82.
Manes TD, Pober JS, Kluger MS. Endothelial cell-T lymphocyte interactions: IP[corrected]-10 stimulates rapid transendothelial migration of human effort but not central memory CD4+ T cells. Requirements for shear stress and adhesion molecules. Transplantation. 2006 Jul 15; 82(1 Suppl):S9-14.
Mayumi M. EoL-1, a human eosinophilic cell line. Leuk. Lymphoma. 1992 Jun; 7(3):243-50.
Ohtsu H, Yamauchi K, Yoshie O, Tanno Y, Saito H, Hayashi N, Takishima T. The effect of cytokines on the differentiation of an eosinophilic leukemia cell line (EoL-1) is associated with down regulation of c-myc gene expression. Cell Struct. Funct. 1993 Apr; 18(2):125-33.
Pande G. The role of membrane lipids in regulation of integrin functions. Curr. Opin. Cell Biol. 2000 Oct; 12(5):569-74.
Pauly A. Brignole-Baudouin F, Guenoun JM, Riancho L, Rat P, Wamet JM, Baudouin C. Comparative study of topical anti-allergic eye drops on human conjunctiva-derived cells: responses to histamine and IFN-γ and toxicological profiles. Graefes Arch. Clin. Exp. Ophthalmol. 2007 Apr; 245(4):534-546. Epub 2006 Aug 10.
Puxeddu I, Ribatti D, Crivellato E, Levi-Schaffer F. Mast cells and eosinophils: a novel link between inflammation and angiogenesis in allergic diseases. J Allergy Clin. Immunol. Sep 2005: 531-536.
Saito H, Bourinbaiar A, Ginsburg M, Minato K, Ceresi E, Yamada K, Machover D, Breard J, Mathe G. Establishment and characterization of a new human eosinophilic leukemia cell line. Blood. 1985 Dec; 66(6):1233-40.
Silvestri M, Bontempelli M, Giacometti M, Malerba M, Rossi GA, Di Stefano A, Rossi A, Ricciardolo FLM. High serum levels of tumour necrosis factor-α and interleukin-8 in severe asthma: markers of systemic inflammation? Clin. and Exp. Allergy 2005; 36: 1373-1381.
Solomon A, Puxeddu I, Levi-Schaffer F. Fibrosis in ocular allergic inflammation: recent concepts in the pathogenesis of ocular allergy. Curr. Opn. Allergy Clin. Immunol. 2003; 3:389-393.
Solorzano C, Bouquelet S, Pereyra MA, Blanco-Favela F, Slomianny MC, Chavez R, Lascurain R, Zenteno E, Agundis C., Isolation and characterization of the potential receptor for wheat germ agglutinin from human neutrophils. Glycoconj J. 2006 Nov; 23(7-8):591-8.
Steube KG, Meyer C, Drexler HG. Induction and secretion of the chemokines interleukin-8 and monocyte chemotactic protein-1 in human immature leukemia cell lines. Mol. Cell Biol. Res. Commun. 2000 Jan; 3(1):60-5.
Strath M.. Warren D.J.. Sanderson C.J., Detection of eosinophils using an eosinophil peroxidase assay. Its use as an assay for eosinophil differentiation factors. J. Immunol. Methods 1985: 83:209-215.
Wen H, Hogaboam CM, Gauldie J, Kunkel SL. Severe sepsis exacerbates cell-mediated immunity in the lung due to an altered dendritic cell cytokine profile. Am. J. Pathol. 2006; 168: 1940-1950.
Wong CK, Cheung PFY, Ip WK, Lam CWK. Intracellular signalling mechanisms regulating Toll-like receptor-mediated activation of eosinophil. Am. J. Respir. Cell Mol. Biol. 2007; 37:85-96.
Zimmermann N, Daugherty BL, Stark JM, Rothenberg ME. Molecular analysis of CCR-3 events in eosinophilic cells. J. Immunol. 2000 Jan 15; 164(2):1055-64.

Various aspects disclosed herein are summarized in the following.
1. A composition comprising: (a) levocabastine or a pharmaceutically acceptable salt or ester thereof; and (b) an additional H₁-receptor antagonist.
2. The composition of aspect 1, wherein the additional H₁-receptor antagonist is selected from the group consisting of acrivastine, cetirizine, azelastine, loratadine, desloratadine, ebastine, mizolastine, fexofenadine, olopatadine, ketotifen, salts thereof, esters thereof, and combinations thereof.
3. The composition of aspect 1, wherein the additional H₁-receptor antagonist is desloratadine.
4. The composition of aspect 1, wherein the additional H₁-receptor antagonist is fexofenadine.
5. The composition of aspect 1, wherein the additional H₁-receptor antagonist is olopatadine.
6. The composition of aspect 1, wherein the additional H₁-receptor antagonist is cetirizine.
7. The composition of aspect 1, wherein the additional H₁-receptor antagonist is ebastine.
8. The composition of aspect 1, wherein the additional H₁-receptor antagonist is ketotifen.
9. The composition of aspect 1, wherein levocabastine or a pharmaceutically acceptable salt or ester thereof, and the additional H₁-receptor antagonist each is independently present at a concentration from about 0.001 mg/ml to about 100 mg/ml.

## Claims

1. A pharmaceutical composition comprising: (a) an active pharmaceutical ingredient ("API"); and (b) a pharmaceutically acceptable vehicle; wherein the API consists of: (i) levocabastine or a pharmaceutically acceptable salt or ester thereof; or (ii) levocabastine and an additional H₁-receptor antagonist, or pharmaceutically acceptable salts or esters thereof; for use in the treatment or controlling of an inflammatory ocular disease, condition, or disorder in a patient, said an inflammatory disease, condition, or disorder in a patient, said disease, condition, or disorder being selected from the group consisting of dry eye, anterior uveitis, iritis, iridocyclitis, keratitis, corneal ulcer, corneal edema, sterile corneal infiltrates, anterior scleritis, episcleritis, blepharitis, post-operative (or post-surgical) ocular inflammation, posterior-segment diseases having etiology in inflammation, inflammatory sequelae of an infection, and combinations thereof.

2. The pharmaceutical composition of claim 1 for use according to claim 1, wherein said post-operative (or post-surgical) ocular inflammation results from photorefractive keratectomy, cataract removal surgery, intraocular lens ("IOL") implantation, laser-assisted in situ keratomileusis ("LASIK"), conductive keratoplasty, or radial keratotomy.

3. The pharmaceutical composition of claim 1 for use according to claim 1, wherein said posterior-segment diseases are selected from the group consisting of diabetic retinopathy ("DR"), dry age-related macular degeneration ("AMD"), wet AMD, diabetic macular edema ("DME"), posterior uveitis, optic neuritis, inflammatory optic neuropathy, optic neuropathy caused by glaucoma, and combinations thereof.

4. The pharmaceutical composition of claim 1 for use according to claim 1, wherein the additional H₁-receptor antagonist is selected from the group consisting of acrivastine, cetirizine, azelastine, loratadine, desloratadine, ebastine, mizolastine, fexofenadine, olopatadine, ketotifen, salts thereof, esters thereof, and combinations thereof.

5. The composition of claim 1 for use according to claim 1, wherein the additional H₁-receptor antagonist is desloratadine.

6. The composition of claim 1 for use according to claim 1, wherein the additional H₁-receptor antagonist is fexofenadine.

7. The composition of claim 1 for use according to claim 1, wherein the additional H₁-receptor antagonist is olopatadine.

8. The composition of claim 1 for use according to claim 1, wherein the additional H₁-receptor antagonist is cetirizine.

9. The composition of claim 1 for use according to claim 1, wherein the additional H₁-receptor antagonist is ebastine.

10. The composition of claim 1 for use according to claim 1, wherein the additional H₁-receptor antagonist is ketotifen.

11. The composition of claim 1 for use according to claim 1, wherein levocabastine, the additional H₁-receptor antagonist, or a pharmaceutically acceptable salt or ester thereof each is independently present at a concentration from about 0.001 mg/ml to about 100 mg/ml.

12. Use of an active pharmaceutical ingredient consisting of: (a) levocabastine or a pharmaceutically acceptable salt or ester thereof; or (b) levocabastine and an additional H₁-receptor antagonist, or pharmaceutically acceptable salts or esters thereof; for the preparation of a medicament for the treatment or control of an inflammatory ocular disease, condition, or disorder in a patient, said disease, condition, or disorder being selected from the group consisting of dry eye, anterior uveitis, iritis, iridocyclitis, keratitis, corneal ulcer, corneal edema, sterile corneal infiltrates, anterior scleritis, episcleritis, blepharitis, post-operative (or post-surgical) ocular inflammation, posterior-segment diseases having etiology in inflammation, inflammatory sequelae of an infection, and combinations thereof.

13. The use of claim 12, wherein said post-operative (or post-surgical) ocular inflammation results from photorefractive keratectomy, cataract removal surgery, intraocular lens ("IOL") implantation, laser-assisted in situ keratomileusis ("LASIK"), conductive keratoplasty, or radial keratotomy.

14. The use of claim 12, wherein said posterior-segment diseases are selected from the group consisting of diabetic retinopathy ("DR"), dry age-related macular degeneration ("AMD"), wet AMD, diabetic macular edema ("DME"), posterior uveitis, optic neuritis, inflammatory optic neuropathy, optic neuropathy caused by glaucoma, and combinations thereof.

15. The use of claim 12, wherein the additional H₁-receptor antagonist is selected from the group consisting of acrivastine, cetirizine, azelastine, loratadine, desloratadine, ebastine, mizolastine, fexofenadine, olopatadine, ketotifen, salts thereof, esters thereof, and combinations thereof.

16. The use of claim 12, wherein the additional H₁-receptor antagonist is desloratadine.

17. The use of claim 12, wherein the additional H₁-receptor antagonist is fexofenadine.

18. The use of claim 12, wherein the additional H₁-receptor antagonist is olopatadine.

19. The use of claim 12, wherein the additional H₁-receptor antagonist is cetirizine.

20. The use of claim 12, wherein the additional H₁-receptor antagonist is ebastine.

21. The use of claim 12, wherein the additional H₁-receptor antagonist is ketotifen.

22. The use of claim 12, wherein levocabastine, the additional H₁-receptor antagonist, or a pharmaceutically acceptable salt or ester thereof each is independently present at a concentration from about 0.001 mg/ml to about 100 mg/ml.

23. The use of claim 12, wherein said disease, condition, or disorder is dry eye.

24. The use of claim 12, wherein said disease, condition, or disorder is keratitis.

25. The use of claim 12, wherein said disease, condition, or disorder is post-operative (or post-surgical) ocular inflammation.

## Patentansprüche

1. Eine pharmazeutische Zusammensetzung umfassend: (a) einen aktiven pharmazeutischen Inhaltsstoff ("API") und (b) einen pharmazeutisch zulässigen Träger, wobei der API aus: (i) Levocabastin oder einem pharmazeutisch zulässigem Salz oder Ester davon oder (ii) Levocabastin und einem zusätzlichen H₁-Rezeptor Antagonisten oder einem pharmazeutisch zulässigem Salz oder Ester davon besteht; zur Verwendung bei der Behandlung oder Kontrolle einer entzündlichen okularen Krankheit, Beschwerde oder Fehlfunktion in einem Patienten, wobei besagte Krankheit, Beschwerde oder Fehlfunktion ausgewählt ist aus der Gruppe bestehend aus trockenen Augen, vorderer Uveitis, Iritis, Iridozyklitis, Keratitis, Korneaulzera, Korneaödemen, sterilen Korneainfiltraten, vorderer Skleritis, Episkleritis, Blepharitis, post-operativen (oder post-chirurgischen) okularen Entzündungen, Erkrankungen des hinteren Augenabschnitts deren Ätiologie eine Entzündung ist, entzündlichen Spätfolgen einer Infektion und Kombinationen daraus.

2. Die pharmazeutische Zusammensetzung gemäß Anspruch 1 zur Verwendung gemäß Anspruch 1, wobei besagte post-operative (oder post-chirurgische) okulare Entzündung aus photorefraktiver Keratektomie, chirurgischer Kataraktentfernung, intraokularer Linsenimplantation ("IOL"), Laser-unterstützter in situ Keratomileusis ("LASIK"), konduktiver Keratoplastie oder radialer Keratotomie resultieren.

3. Die pharmazeutische Zusammensetzung gemäß Anspruch 1 zur Verwendung gemäß Anspruch 1, wobei besagte Erkrankungen des hinteren Augenabschnitts ausgewählt sind aus der Gruppe bestehend aus diabetischer Rethinopathie ("DR"), trockener altersbedingter Makuladegeneration ("AMD"), feuchter AMD, diabetischen Makulaödemen ("DME"), hinterer Uveitis, optischer Neuritis, entzündlicher optischer Neuropathie, durch Glaukom verursachter optischer Neuropathie und Kombinationen daraus.

4. Die pharmazeutische Zusammensetzung gemäß Anspruch 1 zur Verwendung gemäß Anspruch 1, wobei der zusätzliche H₁-Rezeptor Antagonist ausgewählt ist aus der Gruppe bestehend aus Acrivastin, Cetirizin, Azelastin, Loratadin, Desloratadin, Ebastin, Mizolastin, Fexofenadin, Olopatadin, Ketotifen, Salzen davon, Estern davon und Kombination daraus.

5. Die Zusammensetzung gemäß Anspruch 1 zur Verwendung gemäß Anspruch 1, wobei der zusätzliche H₁-Rezeptor Antagonist Desloratadin ist.

6. Die Zusammensetzung gemäß Anspruch 1 zur Verwendung gemäß Anspruch 1, wobei der zusätzliche H₁-Rezeptor Antagonist Fexofenadin ist.

7. Die Zusammensetzung gemäß Anspruch 1 zur Verwendung gemäß Anspruch 1, wobei der zusätzliche H₁-Rezeptor Antagonist Olopatadin ist.

8. Die Zusammensetzung gemäß Anspruch 1 zur Verwendung gemäß Anspruch 1, wobei der zusätzliche H₁-Rezeptor Antagonist Cetirizin ist.

9. Die Zusammensetzung gemäß Anspruch 1 zur Verwendung gemäß Anspruch 1, wobei der zusätzliche H₁-Rezeptor Antagonist Ebastin ist.

10. Die Zusammensetzung gemäß Anspruch 1 zur Verwendung gemäß Anspruch 1, wobei der zusätzliche H₁-Rezeptor Antagonist Ketotifen ist.

11. Die Zusammensetzung gemäß Anspruch 1 zur Verwendung gemäß Anspruch 1, wobei Levocabastin, der zusätzliche H₁-Rezeptor Antagonist oder ein pharmazeutisch zulässiges Salz oder Ester davon jeweils unabhängig voneinander in einer Menge von etwa 0,001 mg/ml bis etwa 100 mg/ml vorhanden sind.

12. Die Verwendung eines aktiven pharmazeutischen Inhaltsstoffs bestehend aus: (a) Levocabastin oder einem pharmazeutisch zulässigem Salz oder Ester davon oder (b) Levocabastin und einem zusätzlichen H₁-Rezeptor Antagonisten oder einem pharmazeutisch zulässigem Salz oder Ester davon; zur Herstellung eines Medikaments zur Behandlung oder Kontrolle einer entzündlichen okularen Krankheit, Beschwerde oder Fehlfunktion in einem Patienten, wobei besagte Krankheit, Beschwerde oder Fehlfunktion ausgewählt ist aus der Gruppe bestehend aus trockenen Augen, vorderer Uveitis, Iritis, Iridozyklitis, Keratitis, Korneaulzera, Korneaödemen, sterilen Korneainfiltraten, vorderer Skleritis, Episkleritis, Blepharitis, post-operativen (oder post-chirurgischen) okularen Entzündungen, Erkrankungen des hinteren Augenabschnitts deren Ätiologie eine Entzündung ist, entzündlichen Spätfolgen einer Infektion und Kombinationen daraus.

13. Die Verwendung gemäß Anspruch 12, wobei besagte post-operative (oder post-chirurgische) okulare Entzündung aus photorefraktiver Keratektomie, chirurgischer Kataraktentfernung, intraokularer Linsenimplantation ("IOL"), Laser-unterstützter in situ Keratomileusis ("LASIK"), konduktiver Keratoplastie oder radialer Keratotomie resultieren.

14. Die Verwendung gemäß Anspruch 12, wobei besagte Erkrankungen des hinteren Augenabschnitts ausgewählt sind aus der Gruppe bestehend aus diabetischer Rethinopathie ("DR"), trockener altersbedingter Makuladegeneration ("AMD"), feuchter AMD, diabetischen Makulaödemen ("DME"), hinterer Uveitis, optischer Neuritis, entzündlicher optischer Neuropathie, durch Glaukom verursachter optischer Neuropathie und Kombinationen daraus.

15. Die Verwendung gemäß Anspruch 12, wobei der zusätzliche H₁-Rezeptor Antagonist ausgewählt ist aus der Gruppe bestehend aus Acrivastin, Cetirizin, Azelastin, Loratadin, Desloratadin, Ebastin, Mizolastin, Fexofenadin, Olopatadin, Ketotifen, Salzen davon, Estern davon und Kombination daraus.

16. Die Verwendung gemäß Anspruch 12, wobei der zusätzliche H₁-Rezeptor Antagonist Desloratadin ist.

17. Die Verwendung gemäß Anspruch 12, wobei der zusätzliche H₁-Rezeptor Antagonist Fexofenadin ist.

18. Die Verwendung gemäß Anspruch 12, wobei der zusätzliche H₁-Rezeptor Antagonist Olopatadin ist.

19. Die Verwendung gemäß Anspruch 12, wobei der zusätzliche H₁-Rezeptor Antagonist Cetirizin ist.

20. Die Verwendung gemäß Anspruch 12, wobei der zusätzliche H₁-Rezeptor Antagonist Ebastin ist.

21. Die Verwendung gemäß Anspruch 12, wobei der zusätzliche H₁-Rezeptor Antagonist Ketotifen ist.

22. Die Verwendung gemäß Anspruch 12, wobei Levocabastin, der zusätzliche H₁-Rezeptor Antagonist oder ein pharmazeutisch zulässiges Salz oder Ester davon jeweils unabhängig voneinander in einer Menge von etwa 0,001 mg/ml bis etwa 100 mg/ml vorhanden sind.

23. Die Verwendung gemäß Anspruch 12, wobei besagte Krankheit, Beschwerde oder Fehlfunktion ein trockenes Auge ist.

24. Die Verwendung gemäß Anspruch 12, wobei besagte Krankheit, Beschwerde oder Erkrankung Keratitis ist.

25. Die Verwendung gemäß Anspruch 12, wobei besagte Krankheit, Beschwerde oder Fehlfunktion eine post-operative (oder post-chirurgische) okulare Entzündung ist.

## Revendications

1. Composition pharmaceutique comprenant : (a) un ingrédient pharmaceutique actif (« IPA ») ; et (b) un véhicule pharmaceutiquement acceptable ; dans laquelle l'IPA consiste en : (i) de la lévocabastine ou un sel ou un ester pharmaceutiquement acceptable de celle-ci ; ou (ii) de la lévocabastine et un antagoniste des récepteurs H₁ supplémentaire, ou des sels ou des esters pharmaceutiquement acceptables de ceux-ci ; destinée à être utilisée dans le traitement ou le contrôle d'une maladie, d'un état pathologique ou d'un trouble oculaire inflammatoire chez un patient, ladite maladie, état pathologique ou trouble étant sélectionné(e) dans le groupe consistant en la sécheresse oculaire, l'uvéite antérieure, l'iritis, l'iridocyclite, la kératite, un ulcère cornéen, un oedème cornéen, des infiltrats cornéens stériles, la sclérite antérieure, l'épisclérite, la blépharite, une inflammation oculaire post-opératoire (ou post-chirurgicale), des maladies du segment postérieur ayant une étiologie inflammatoire, des séquelles inflammatoires d'une infection, et des combinaisons de celles-ci.

2. Composition pharmaceutique selon la revendication 1 destinée à être utilisée selon la revendication 1, où ladite inflammation oculaire post-opératoire (ou post-chirurgicale) est le résultat d'une photokératectomie réfractive, d'une chirurgie de la cataracte, de l'implantation d'une lentille intraoculaire (« LIO »), d'une kératomileusie *in situ* assistée par laser (« LASIK »), d'une kératoplastie conductive, ou d'une kératotomie radiale.

3. Composition pharmaceutique selon la revendication 1 destinée à être utilisée selon la revendication 1, où lesdites maladies du segment postérieur sont sélectionnées dans le groupe consistant en la rétinopathie diabétique (« RD »), la dégénérescence maculaire liée à l'âge (DMLA) sèche, la DMLA humide, un oedème maculaire diabétique (« OMD »), une uvéite postérieure, une névrite optique, une neuropathie optique inflammatoire, une neuropathie optique provoquée par un glaucome, et des combinaisons de celles-ci.

4. Composition pharmaceutique selon la revendication 1 destinée à être utilisée selon la revendication 1, dans laquelle l'antagoniste des récepteurs H₁ supplémentaire est sélectionné dans le groupe consistant en l'acrivastine, la cétirizine, l'azélastine, la loratadine, la desloratadine, l'ébastine, la mizolastine, la fexofénadine, l'olopatadine, le kétotifene, des sels de ceux-ci, des esters de ceux-ci, et des combinaisons de ceux-ci.

5. Composition selon la revendication 1 destinée à être utilisée selon la revendication 1, dans laquelle l'antagoniste des récepteurs H₁ supplémentaire est la desloratadine.

6. Composition selon la revendication 1 destinée à être utilisée selon la revendication 1, dans laquelle l'antagoniste des récepteurs H₁ supplémentaire est la fexofénadine.

7. Composition selon la revendication 1 destinée à être utilisée selon la revendication 1, dans laquelle l'antagoniste des récepteurs H₁ supplémentaire est l'olopatadine.

8. Composition selon la revendication 1 destinée à être utilisée selon la revendication 1, dans laquelle l'antagoniste des récepteurs H₁ supplémentaire est la cétirizine.

9. Composition selon la revendication 1 destinée à être utilisée selon la revendication 1, dans laquelle l'antagoniste des récepteurs H₁ supplémentaire est l'ébastine.

10. Composition selon la revendication 1 destinée à être utilisée selon la revendication 1, dans laquelle l'antagoniste des récepteurs H₁ supplémentaire est le kétotifène.

11. Composition selon la revendication 1 destinée à être utilisée selon la revendication 1, dans laquelle la lévocabastine, l'antagoniste des récepteurs H₁ supplémentaire, ou un sel ou un ester pharmaceutiquement acceptable de celle-ci/celui-ci, est chacun indépendamment présent à une concentration comprise entre environ 0,001 mg/ml et environ 100 mg/ml.

12. Utilisation d'un ingrédient pharmaceutique actif consistant en : (a) de la lévocabastine ou un sel ou un ester pharmaceutiquement acceptable de celle-ci ; ou (b) de la lévocabastine et un antagoniste des récepteurs H₁ supplémentaire, ou des sels ou des esters pharmaceutiquement acceptables de ceux-ci ; pour la préparation d'un médicament destiné au traitement ou au contrôle d'une maladie, d'un état pathologique ou d'un trouble oculaire inflammatoire chez un patient, ladite maladie, état pathologique ou trouble étant sélectionné(e) dans le groupe consistant en la sécheresse oculaire, l'uvéite antérieure, l'iritis, l'iridocyclite, la kératite, un ulcère cornéen, un oedème cornéen, des infiltrats cornéens stériles, la sclérite antérieure, l'épisclérite, la blépharite, une inflammation oculaire post-opératoire (ou post-chirurgicale), des maladies du segment postérieur ayant une étiologie inflammatoire, des séquelles inflammatoires d'une infection, et des combinaisons de celles-ci.

13. Utilisation selon la revendication 12, dans laquelle ladite inflammation oculaire post-opératoire (ou post-chirurgicale) est le résultat d'une photokératectomie réfractive, d'une chirurgie de la cataracte, de l'implantation d'une lentille intraoculaire (« LIO »), d'une kératomileusie *in situ* assistée par laser (« LASIK »), d'une kératoplastie conductive, ou d'une kératotomie radiale.

14. Utilisation selon la revendication 12, dans laquelle lesdites maladies du segment postérieur sont sélectionnées dans le groupe consistant en la rétinopathie diabétique (« RD »), la dégénérescence maculaire liée à l'âge (DMLA) sèche, la DMLA humide, un oedème maculaire diabétique (« OMD »), une uvéite postérieure, une névrite optique, une neuropathie optique inflammatoire, une neuropathie optique provoquée par un glaucome, et des combinaisons de celles-ci.

15. Utilisation selon la revendication 12, dans laquelle l'antagoniste des récepteurs H₁ supplémentaire est sélectionné dans le groupe consistant en l'acrivastine, la cétirizine, l'azélastine, la loratadine, la desloratadine, l'ébastine, la mizolastine, la fexofénadine, l'olopatadine, le kétotifène, des sels de ceux-ci, des esters de ceux-ci, et des combinaisons de ceux-ci.

16. Utilisation selon la revendication 12, dans laquelle l'antagoniste des récepteurs H₁ supplémentaire est la desloratadine.

17. Utilisation selon la revendication 12, dans laquelle l'antagoniste des récepteurs H₁ supplémentaire est la fexofénadine.

18. Utilisation selon la revendication 12, dans laquelle l'antagoniste des récepteurs H₁ supplémentaire est l'olopatadine.

19. Utilisation selon la revendication 12, dans laquelle l'antagoniste des récepteurs H₁ supplémentaire est la cétirizine.

20. Utilisation selon la revendication 12, dans laquelle l'antagoniste des récepteurs H₁ supplémentaire est l'ébastine.

21. Utilisation selon la revendication 12, dans laquelle l'antagoniste des récepteurs H₁ supplémentaire est le kétotifène.

22. Utilisation selon la revendication 12, dans laquelle la lévocabastine, l'antagoniste des récepteurs H₁ supplémentaire, ou un sel ou un ester pharmaceutiquement acceptable de celle-ci/celui-ci, est chacun indépendamment présent à une concentration comprise entre environ 0,001 mg/ml et environ 100 mg/ml.

23. Utilisation selon la revendication 12, dans laquelle ladite maladie, état pathologique ou trouble est la sécheresse oculaire.

24. Utilisation selon la revendication 12, dans laquelle ladite maladie, état pathologique ou trouble est la kératite.

25. Utilisation selon la revendication 12, dans laquelle ladite maladie, état pathologique ou trouble est une inflammation oculaire post-opératoire (ou post-chirurgicale).
